(19) Europäisches Patentamt
European Patent Office
Office européen des brevets

(11) EP 0 693 284 A1

(12) EUROPEAN PATENT APPLICATION
published in accordance with Art. 158(3) EPC

(43) Date of publication:
24.01.1996 Bulletin 1996/04

(21) Application number: 95906535.0

(22) Date of filing: 27.01.1995

(51) Int. Cl.$^6$: A61K 31/557

(86) International application number: PCT/JP95/00106

(87) International publication number: WO 95/20391
(03.08.1995 Gazette 1995/33)

(84) Designated Contracting States:
AT BE CH DE ES FR GB IT LI NL SE

(30) Priority: 28.01.1994 JP 8892/94
16.02.1994 JP 19258/94
01.08.1994 JP 180093/94

(71) Applicant: TEIJIN LIMITED
Osaka-shi, Osaka 541 (JP)

(72) Inventors:
- HANAJIMA, Nobuaki
Teijin Limited,
Hino-shi Tokyo 191 (JP)
- OKAMURA, Noriaki
Teijin Limited,
Hino-shi Tokyo 191 (JP)

(74) Representative: Greaves, Carol Pauline et al
London WC2B 6HP (GB)

# (54) PROSTACYCLIN-CONTAINING OLEAGINOUS OINTMENT

(57) A prostacyclin-containing skin-care composition comprises, as the active ingredient, (i) a prostacyclin represented by general formula (I) and/or an enantiomer thereof, wherein $R^1$ represents hydrogen or $C_1$-$C_{10}$ linear or branched alkyl; $R^2$ represents $R^{21}$ or $(CH_2)_nR^{22}$ ($R^{21}$ representing optionally substituted $C_3$-$C_{12}$ linear or branched alkyl, alkenyl or alkynyl, $R^{22}$ representing optionally substituted $C_3$-$C_{10}$ cycloalkyl, and n representing an integer of 0 to 2); and $R^3$ and $R^4$ represent each independently hydrogen or a group represented by general formula (II), wherein $R^5$ represents $C_1$-$C_5$ linear or branched alkyl, (ii) a vaseline, (iii) a paraffin, and (iv) a fatty acid ester, and further contains, if necessary, liquid paraffin. Another composition comprises a prostacyclin represented by the above general formula (I) and/or an enantiomer thereof, a vaseline and a glycol, and further contains, if necessary, a paraffin and/or liquid paraffin.

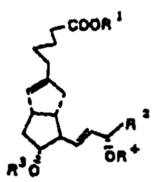

(I)

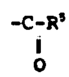

(II)

EP 0 693 284 A1

## Description

### TECHNICAL FIELD

The present invention relates to a skin application composition including a prostacyclin as an active ingredient and superior in duration of medicinal effect, in particular an oily ointment containing a prostacyclin.

### BACKGROUND ART

As medications for the treatment of bed sores, burns, and other various kinds of skin sores, antibiotics aimed at killing bacteria, enzyme preparations aimed at digesting the tissue, skin cleansing solutions, water-absorbing polymer powders, injury covering agents, etc. have been used. In recent years, as a medication with a new mechanism of action, attempts have been made to apply prostaglandins, which have an action improving the peripheral circulation, an action suppressing platelet aggregation, and an action expanding veins, to these skin sores as transdermal absorption type preparations (see Japanese Unexamined Patent Publication (Kokai) No. 63-99866, Japanese Unexamined Patent Publication (Kokai) No. 63-135333, Japanese Unexamined Patent Publication (Kokai) No. 63-146812, Japanese Unexamined Patent Publication (Kokai) No. 1-143340, Japanese Unexamined Patent Publication (Kokai) No. 3-83925, Japanese Unexamined Patent Publication (Kokai) No. 3-83926, Japanese Unexamined Patent Publication (Kokai) No. 4-164034, Japanese Unexamined Patent Publication (Kokai) No. 4-243827, Japanese Unexamined Patent Publication (Kokai) No. 4-300833). Further, it has been shown that the compounds of the following formula (I) and isomers of the same have a superior action in improving peripheral circulation and that external preparations including the same have a pharmacological activity suggesting possibilities as a medication for the treatment of ulcerous skin diseases (see PCT International Publication No. 94/17805).

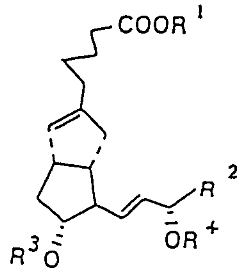

(I)

Preparations for application to the skin include semisolid agents (ointments and creams), plasters, lotions, etc. For application to locations of skin sores caused by peripheral circulatory disorders or locations where there is a chance of skin sores, a low irritating ointment, in particular, an oily ointment is preferred. An oily ointment of a prostaglandin has been reported in Japanese Unexamined Patent Publication (Kokai) No. 4-243827. Further, in transdermal absorption type preparations, glycols are used in almost all cases as promotors of absorption. A report of them being agents for control of the release may be found only in Japanese Unexamined Patent Publication (Kokai) No. 60-13720 and Japanese Unexamined Patent Publication (Kokai) No. 60-161918.

When using a compound having the above formula (I) and isomers thereof as an external preparation, the compound easily is deactivated by the β-oxidation in the body, compared with, for example, the compound described in Japanese Unexamined Patent Publication (Kokai) No. 4-243827, since the 3-position in the prostaglandin nomenclature is not substituted by an oxygen atom. Further, the isomer with the 16-position or 17-position not substituted by an alkyl group is susceptible to oxidation of the hydroxyl group of the 15-position, and therefore, has a short lifetime in the body. Accordingly, the compound having the above formula (I) is extremely highly active, a general characteristic of prostaglandins, and has an activity like that of ootacoids (local hormones), that is, has a short lifetime in the body and has few systemic side effects.

For this reason, when administered as a general external preparation, there is the problem that after being absorbed from the skin location, the compound is quickly metabolized in the body, and therefore, the duration of the action on increasing the blood flow and other pharmacological actions is poor. Accordingly, it is necessary to deliver a sufficient amount of the active ingredient to the skin continuously by properly designing the preparation.

In general, in cases of skin diseases where there are sores, the skin is not keratinous, so when applying an external preparation, in general the active ingredient easily permeates through the skin. On the other hand, even with peripheral circulatory disorders, in the case of Raynaud's disease or Barger's disease where there are no sores, there is keratin at the skin of the location where the medicine is administered, so the absorption is poor. However, prostaglandins are

generally highly active and have a short lifetime in the body. Therefore, when designing an external preparation using a prostaglandin as an active ingredient, it is insufficient to merely increase the amount of transdermal absorption. Making the active ingredient be released from the base constantly is extremely important in preventing side effects. If such control of the release were possible, then the concentration of the active ingredient in the base could be lowered and there would be advantages both in terms of safety and economy.

From this viewpoint, Japanese Unexamined Patent Publication (Kokai) No. 4-243827 shows an example of an oily ointment having a good stability, but this publication has no description at all of the duration of the medicinal effect. In fact, when following the method of the examples to prepare an external preparation of a prostacyclin having the above formula (I), it was not possible to control the duration of the medicinal effect or the release.

There is a report, however, showing that with salicylic acid etc., when normal paraffin is mixed with vaseline, the viscosity rises and the rate of diffusion of the medicine falls (see Pharm. Ind. vol. 48, pp. 969-972, 1986). Further, there is a report that the duration of a gentamicin ointment of a ratio of vaseline and paraffin of 95:5 is good (see Antibiotiki,vol. 23, pp, 257-259, 1978).

However, in these prior art, no description was made at all of the important role played by the fatty acid ester, that is, the solubilizing agent, in relation to the prolongation of the duration of the pharmacological effect of the prostaglandins of the oily ointment of the present invention and therefore cannot be said to have been technically applicable.

On the other hand, the above two prior art relating to glycols made direct use of the solvent, absorption promoter, and glycol solution, and therefore, are art unrelated to an oily ointment.

DISCLOSURE OF THE INVENTION

Accordingly, the problem to be solved by the present invention is to provide a preparation, for use when making a prostacyclin into an external preparation, which is superior in transdermal absorption and controls the release of the active ingredient from the base so that it can be safely used at diseased portions of the skin regardless of the presence or absence of keratin.

Accordingly, the present inventors engaged in repeated intensive studies to achieve the above problem and, as a result, found that by using vaseline, a suitable amount of paraffin, and a solubilizing agent, it is possible to control the release of the active ingredient from an ointment, and thereby succeeded in obtaining an oily ointment with an excellent transdermal absorption and duration. Further, they found that by blending into the vaseline a suitable amount of a glycol, it is possible to obtain a superior effect of controlling the release, found an oily ointment superior in the transdermal absorption and duration of medicinal effect of the prostaglandin of the above formula (I), whereby the present invention is reached.

That is, in accordance with the present invention, there is provided a prostacyclin-containing skin application composition comprising, as a medicinally active component, a prostacyclin having the formula (I)

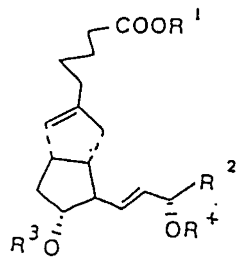

(I)

wherein, $R^1$ represents a hydrogen atom, $C_1$ to $C_{10}$ straight or branched alkyl group, $R^2$ represents $R^{21}$ or $(CH_2)_nR^{22}$ where, $R^{21}$ is a $C_3$ to $C_{12}$ straight or branched alkyl, alkenyl or alkynyl group which may be substituted, $R^{22}$ represents a $C_3$ to $C_{10}$ cycloalkyl group which may be substituted, n is an integer of 0 to 2, $R^3$ and $R^4$ are each independently hydrogen atom or formula (II)

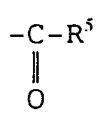

(II)

wherein, $R^5$ is a $C_1$ to $C_5$ straight or branched alkyl group and/or its enantiomers, in a first aspect of the present invention, a vaseline, paraffin, and fatty acid ester and, optionally or if desired liquid paraffin.

In a second aspect of the present invention, there is provided a prostacyclin-containing skin application composition which contains the above prostacyclin and/or its enantiomers, vaseline, and a glycol and, optionally, a paraffin and/or liquid paraffin.

BRIEF DESCRIPTION OF THE DRAWINGS

The present invention will now be explained in detail with reference to the drawings, wherein:

Fig. 1 is a graph of the present invention showing the results of an experiment on the release of the active ingredient from the preparation,
Fig. 2 is a graph of the present invention showing the results of an experiment on the release of the active ingredient from the preparation,
Fig. 3 is a graph of the present invention showing the results of an experiment on the release of the active ingredient from the preparation,
Fig. 4 is a graph which shows the results of a test on the amount of blood flow at the skin in local application of the preparation of the present invention,
Fig. 5 is a graph which shows the results of a test on the amount of blood flow at the skin in local application of the preparation of the present invention,
Fig. 6 is a graph which shows the results of a test on the amount of blood flow at the skin in local application of the preparation of the present invention, and
Fig. 7 is a graph which shows the results of a test on the amount of blood flow at the skin in local application of the preparation of the present invention.

BEST MODE FOR CARRYING OUT THE INVENTION

In the above formula (I), $R^1$ is a hydrogen atom or $C_1$ to $C_{10}$ straight or branched alkyl group. As the $C_1$ to $C_{10}$ alkyl group, for example, a methyl group, ethyl group, n-propyl group, iso-propyl group, n-butyl group, sec-butyl group, tert-butyl group, n-pentyl group, n-hexyl group, n-heptyl group, n-octyl group, n-nonyl group, n-decyl group, etc. may be mentioned. Among these, a $C_1$ to $C_6$ alkyl group, in particular a $C_1$ to $C_4$ alkyl group, is preferred. As the $R^1$, a hydrogen atom or a methyl group is preferable, in particular a methyl group is preferable.

In the above formula (I), $R^2$ is $R^{21}$ or $(CH_2)_nR^{22}$ where, $R^{21}$ is a $C_3$ to $C_{12}$ straight or branched alkyl, alkenyl, or alkynyl group, which may be substituted, $R^{22}$ is a $C_3$ to $C_{10}$ cycloalkyl group which may be substituted, and n is an integer of 0 to 2. As the unsubstituted $C_3$ to $C_{12}$ alkyl group relating to $R^{21}$, for example, the n-propyl group, n-butyl group, n-pentyl group, 3-methylbutyl group, n-hexyl group, 4-methylpentyl group, 3,3-dimethylbutyl group, n-heptyl group, 5-methylhexyl group, 4-methylhexyl group, 3-methylhexyl group, n-octyl group, 6-methylheptyl group, 5-methylheptyl group, 4-methylheptyl group, 3-methylheptyl group, n-nonyl group, 7-methyloctyl group, 6-methyloctyl group, 5-methyloctyl group, 4-methyloctyl group, 3-methyloctyl group, iso-propyl group, 2-methylbutyl group, 2-methylpentyl group, 2,3-dimethylbutyl group, 2-methylhexyl group, 2,4-dimethylpentyl group, 2,2,3-trimethylbutyl group, 2-methylheptyl group, 2,5-dimethylhexyl group, 2,4-dimethylhexyl group, 2,5-dimethylhexyl group, 2-methyloctyl group, 2,6-dimethylheptyl group, 2,5-dimethylheptyl group, 2,4-dimethylheptyl group, 2,3-dimethylheptyl group, 2-methylnonyl group, 2,7-dimethyloctyl group, 2,6-dimethyloctyl group, 2,5-dimethyloctyl group, 2,4-dimethyloctyl group, 2,3-dimethyloctyl group, etc. may be mentioned. Among these, a $C_3$ to $C_{10}$ alkyl group, in particular a $C_5$ to $C_{10}$ alkyl group are preferred.

Further, as the unsubstituted $C_3$ to $C_{12}$ alkenyl group relating to $R^{21}$, for example, 3-butenyl group, 2-methyl-3-butenyl group, 3-pentenyl group, 2-methyl-3-pentenyl group, 4-pentenyl group, 2-methyl-4-pentenyl group, 2,3-dimethyl-4-pentenyl group, 3-methyl-4-pentenyl group, 3-ethyl-2-methyl-4-pentenyl group, 3-hexenyl group, 4-hexenyl group, 5-hexenyl group, 2-methyl-3-hexenyl group, 2-methyl-4-hexenyl group, 2-methyl-5-hexenyl group, 5-methyl-3-hexenyl group, 5,5-dimethyl-hexenyl group, 2,5-dimethyl-3-hexenyl group, 2,3-dimethyl-4-hexenyl group, 4-methyl-5-hexenyl group, 2,4-dimethyl-5-hexenyl group, 4,4-dimethyl-5-hexenyl group, 2,4,4-trimethyl-5-hexenyl group, 3-methyl-5-hexenyl group, 2,3-dimethyl-5-hexenyl group, 3-heptenyl group, 4-heptenyl group, 5-heptenyl group, 6-heptenyl group, 2-methyl-3-heptenyl group, 2-methyl-4-heptenyl group, 2-methyl-5-heptenyl group, 2-methyl-6-heptenyl group, 5-methyl-6-heptenyl group, 2,5-dimethyl-6-heptenyl group, 5,5-dimethyl-6-heptenyl group, 2,5,5-trimethyl-6-heptenyl group, 5-ethyl-6-heptenyl group, 5-ethyl-2-methyl-6-heptenyl group, 4-methyl-6-heptenyl group, 2,4-dimethyl-6-heptenyl group, 4,4-dimethyl-6-heptenyl group, 2,4,4-trimethyl-6-heptenyl group, 3-methyl-6-heptenyl group, 2,3-dimethyl-6-heptenyl group, 6-methyl-4-heptenyl group, 2,6-dimethyl-4-heptenyl group, 6,6-dimethyl-4-heptenyl group, 2,6,6-trimethyl-4-heptenyl group, 3-methyl-5-heptenyl group, 2,3-dimethyl-5-heptenyl group, 3-methyl-4-heptenyl group, 5-methyl-3-heptenyl group, 2,5-dimethyl-3-heptenyl group, 3-octenyl group, 4-octenyl group, 5-octenyl group, 6-octenyl group, 7-octenyl group, 2-methyl-3-octenyl group, 2-methyl-4-octenyl group, 2-methyl-5-octenyl group, 2-methyl-6-octenyl group, 2-methyl-7-octenyl group, 6-methyl-7-octenyl group, 2,6-dimethyl-7-octenyl group, 6,6-dimethyl-7-octenyl group, 2,6,6-trimethyl-7-octenyl group, 5-methyl-7-octenyl group, 2,5-dimethyl-7-octenyl group, 4-methyl-7-octenyl group, 2,4-dimethyl-7-octenyl group, 4,4-dimethyl-7-octenyl group, 2,4,4-trimethyl-7-octenyl group, 3-methyl-7-octenyl group, 2,3-dimethyl-7-octenyl group, 3,3-dimethyl-7-octenyl group, 2,3,3-trimethyl-7-octenyl group, 3-methyl-6-octenyl group, 2,3-dimethyl-6-octenyl group, 4-methyl-6-octenyl group, 2,4-dimethyl-6-octenyl group, 4,4-dimethyl-6-octenyl group, 2,4,4-trimethyl-6-octenyl group, 5-methyl-6-octenyl group, 2,5-dimethyl-6-octenyl group, 5,5-dimethyl-6-octenyl group, 2,5,5-trimethyl-6-octenyl group, 7-methyl-5-octenyl group, 2,7-dimethyl-5-octenyl group, 4-methyl-5-octenyl group, 2,4-dimethyl-5-octenyl group, 4,4-dimethyl-5-octenyl group, 2,4,4-trimethyl-5-octenyl group, 7,7-dimethyl-5-octenyl group, 2,7,7-trimethyl-5-octenyl group, 6-methyl-4-octenyl group, 2,6-dimethyl-4-octenyl group, 6-ethyl-4-octenyl group, 6-ethyl-2-methyl-4-octenyl group, 6,6-dimethyl-4-octenyl group, 2,6,6-trimethyl-4-octenyl group, 5-methyl-3-octenyl group, 2,5-dimethyl-3-octenyl group, 6-methyl-3-octenyl group, 2,6-dimethyl-3-octenyl group, 7-methyl-3-octenyl group, 2,7-dimethyl-3-octenyl group, 3-nonenyl group, 4-nonenyl group, 5-nonenyl group, 6-nonenyl group, 7-nonenyl group, 8-nonenyl group, 2-methyl-3-nonenyl group, 2-methyl-4-nonenyl group, 2-methyl-5-nonenyl group, 2-methyl-6-nonenyl group, 2-methyl-7-nonenyl group, 2-methyl-8-nonenyl group, 7-methyl-8-nonenyl group, 2,7-dimethyl-8-nonenyl group, 7,7-dimethyl-8-nonenyl group, 2,7,7-trimethyl-8-nonenyl group, 6-methyl-8-nonenyl group, 2,6-dimethyl-8-nonenyl group, 6,6-dimethyl-8-nonenyl group, 2,6,6-trimethyl-8-nonenyl group, 5-methyl-8-nonenyl group, 2,5-dimethyl-8-nonenyl group, 5,5-dimethyl-8-nonenyl group, 2,5,5-trimethyl-8-nonenyl group, 4-methyl-8-nonenyl group, 2,4-dimethyl-8-nonenyl group, 5-ethyl-8-nonenyl group, 5-ethyl-2-methyl-8-nonenyl group, 3-methyl-8-nonenyl group, 2,3-dimethyl-8-nonenyl group, 2,3,3-trimethyl-8-nonenyl group, 3-methyl-7-nonenyl group, 2,3-dimethyl-7-nonenyl group, 3,3-dimethyl-7-nonenyl group, 2,3,3-trimethyl-7-nonenyl group, 4-methyl-7-nonenyl group, 2,4-dimethyl-7-nonenyl group, 4,4-dimethyl-7-nonenyl group, 5-methyl-7-nonenyl group, 2,5-dimethyl-7-nonenyl group, 5,5-dimethyl-7-nonenyl group, 2,5,5-trimethyl-7-nonenyl group, 6-methyl-7-nonenyl group, 2,6-dimethyl-7-nonenyl group, 6,6-dimethyl-7-nonenyl group, 2,6,6-trimethyl-7-nonenyl group, 3-methyl-6-nonenyl group, 2,3-dimethyl-6-nonenyl group, 4-methyl-6-nonenyl group, 2,4-dimethyl-6-nonenyl group, 4,4-dimethyl-6-nonenyl group, 2,4,4-trimethyl-6-nonenyl group, 5-methyl-6-nonenyl group, 2,5-dimethyl-6-nonenyl group, 8-methyl-6-nonenyl group, 2,8-dimethyl-6-nonenyl group, 8,8-dimethyl-6-nonenyl group, 2,8,8-trimethyl-6-nonenyl group, 3-methyl-5-nonenyl group, 2,3-dimethyl-5-nonenyl group, 3,3-dimethyl-5-nonenyl group, 2,3,3-trimethyl-5-nonenyl group, 4-methyl-5-nonenyl group, 2,4-dimethyl-5-nonenyl group, 4,4-dimethyl-5-nonenyl group, 2,4,4-trimethyl-5-nonenyl group, 7-methyl-5-nonenyl group, 2,7-dimethyl-5-nonenyl group, 7-ethyl-5-nonenyl group, 7-ethyl-2-methyl-5-nonenyl group, 8-methyl-5-nonenyl group, 2,8-dimethyl-5-nonenyl group, 8,8-dimethyl-5-nonenyl group, 2,8,8-trimethyl-5-nonenyl group, 3-methyl-4-nonenyl group, 2,3-dimethyl-4-nonenyl group, 6-methyl-4-nonenyl group, 2,6-dimethyl-4-nonenyl group, 6,6-dimethyl-4-nonenyl group, 2,6,6-trimethyl-4-nonenyl group, 7,7-dimethyl-4-nonenyl group, 2,7,7-trimethyl-4-nonenyl group, 7-ethyl-4-nonenyl group, 7-ethyl-2-methyl-4-nonenyl group, etc. may be mentioned. Among these, a $C_4$ to $C_{10}$ alkenyl group, in particular a $C_5$ to $C_{10}$ alkenyl group is preferred.

Further, as the unsubstituted $C_3$ to $C_{12}$ alkynyl group relating to $R^{21}$, for example, 3-butynyl group, 2-methyl-3-butynyl group, 3-pentynyl group, 2-methyl-3-pentynyl group, 4-pentynyl group, 2-methyl-4-pentynyl group, 2,3-dimethyl-4-pentynyl group, 3-methyl-4-pentynyl group, 3-ethyl-2-methyl-4-pentynyl group, 3-hexynyl group, 4-hexynyl group, 5-hexynyl group, 2-methyl-3-hexynyl group, 2-methyl-4-hexynyl group, 2-methyl-5-hexynyl group, 5-methyl-3-hexynyl group, 5,5-dimethyl-hexynyl group, 2,5-dimethyl-3-hexynyl group, 2,3-dimethyl-4-hexynyl group, 4-methyl-5-hexynyl group, 2,4-dimethyl-5-hexynyl group, 4,4-dimethyl-5-hexynyl group, 2,4,4-trimethyl-5-hexynyl group, 3-methyl-5-hexynyl group, 2,3-dimethyl-5-hexynyl group, 3-heptynyl group, 4-heptynyl group, 5-heptynyl group, 6-heptynyl group, 2-methyl-3-heptynyl group, 2-methyl-4-heptynyl group, 2-methyl-5-heptynyl group, 2-methyl-6-heptynyl group, 5-methyl-6-heptynyl group, 2,5-dimethyl-6-heptynyl group, 5,5-dimethyl-6-heptynyl group, 2,5,5-trimethyl-6-heptynyl group, 5-ethyl-6-heptynyl group, 5-ethyl-2-methyl-6-heptynyl group, 4-methyl-6-heptynyl group, 2,4-dimethyl-6-heptynyl group, 4,4-dimethyl-6-heptynyl group, 2,4,4-trimethyl-6-heptynyl group, 3-methyl-6-heptynyl group, 2,3-dimethyl-6-heptynyl group, 6-methyl-4-heptynyl group, 2,6-dimethyl-4-heptynyl group, 6,6-dimethyl-4-heptynyl group, 2,6,6-trimethyl-4-heptynyl group, 3-methyl-5-heptynyl group, 2,3-dimethyl-5-heptynyl group, 3-methyl-4-heptynyl group, 5-methyl-3-heptynyl group, 2,5-dimethyl-3-heptynyl group, 3-octynyl group, 4-octynyl group, 5-octynyl group, 6-octynyl group, 7-octynyl group, 2-methyl-3-octynyl group, 2-methyl-4-octynyl group, 2-methyl-5-octynyl group, 2-methyl-6-octynyl group, 2-methyl-7-octynyl group, 6-methyl-7-octynyl group, 2,6-dimethyl-7-octynyl group, 6,6-dimethyl-7-octynyl group, 2,6,6-trimethyl-7-octynyl group, 5-methyl-7-octynyl group, 2,5-dimethyl-7-octynyl group, 4-methyl-7-octynyl group, 2,4-dimethyl-7-octynyl group, 4,4-dimethyl-7-octynyl group, 2,4,4-trimethyl-7-octynyl group, 3-methyl-7-octynyl group, 2,3-dimethyl-7-octynyl group, 3,3-dimethyl-7-octynyl group, 2,3,3-trimethyl-7-octynyl group, 3-methyl-6-octynyl group, 2,3-dimethyl-6-octynyl group, 4-methyl-6-octynyl group, 2,4-dimethyl-6-octynyl group, 4,4-dimethyl-6-octynyl group, 2,4,4-trimethyl-6-octynyl group, 5-methyl-6-octynyl group, 2,5-dimethyl-6-octynyl group, 5,5-dimethyl-6-octynyl group, 2,5,5-trimethyl-6-octynyl group, 7-methyl-5-octynyl group, 2,7-dimethyl-5-octynyl group, 4-methyl-5-octynyl group, 2,4-dimethyl-5-octynyl group, 4,4-dimethyl-5-octynyl group, 2,4,4-trimethyl-5-octynyl group, 7,7-dimethyl-5-octynyl group, 2,7,7-trimethyl-5-octynyl group, 6-methyl-4-octynyl group, 2,6-dimethyl-4-octynyl group, 6-ethyl-4-octynyl group, 6-ethyl-2-methyl-4-octynyl group, 6,6-dimethyl-4-octynyl group, 2,6,6-trimethyl-4-octynyl group, 5-methyl-3-octynyl group, 2,5-dimethyl-3-octynyl group, 6-methyl-3-octynyl group, 2,6-dimethyl-3-octynyl group, 7-methyl-3-octynyl group, 2,7-dimethyl-3-octynyl group, 3-nonynyl group,

4-nonynyl group, 5-nonynyl group, 6-nonynyl group, 7-nonynyl group, 8-nonynyl group, 2-methyl-3-nonynyl group, 2-methyl-4-nonynyl group, 2-methyl-5-nonynyl group, 2-methyl-6-nonynyl group, 2-methyl-7-nonynyl group, 2-methyl-8-nonynyl group, 7-methyl-8-nonynyl group, 2,7-dimethyl-8-nonynyl group, 7,7-dimethyl-8-nonynyl group, 2,7,7-trimethyl-8-nonynyl group, 6-methyl-8-nonynyl group, 2,6-dimethyl-8-nonynyl group, 6,6-dimethyl-8-nonynyl group, 2,6,6-trimethyl-8-nonynyl group, 5-methyl-8-nonynyl group, 2,5-dimethyl-8-nonynyl group, 5,5-dimethyl-8-nonynyl group, 2,5,5-trimethyl-8-nonynyl group, 4-methyl-8-nonynyl group, 2,4-dimethyl-8-nonynyl group, 5-ethyl-8-nonynyl group, 5-ethyl-2-methyl-8-nonynyl group, 3-methyl-8-nonynyl group, 2,3-dimethyl-8-nonynyl group, 2,3,3-trimethyl-8-nonynyl group, 3-methyl-7-nonynyl group, 2,3-dimethyl-7-nonynyl group, 3,3-dimethyl-7-nonynyl group, 2,3,3-trimethyl-7-nonynyl group, 4-methyl-7-nonynyl group, 2,4-dimethyl-7-nonynyl group, 4,4-dimethyl-7-nonynyl group, 5-methyl-7-nonynyl group, 2,5-dimethyl-7-nonynyl group, 5,5-dimethyl-7-nonynyl group, 2,5,5-trimethyl-7-nonynyl group, 6-methyl-7-nonynyl group, 2,6-dimethyl-7-nonynyl group, 6,6-dimethyl-7-nonynyl group, 2,6,6-trimethyl-7-nonynyl group, 3-methyl-6-nonynyl group, 2,3-dimethyl-6-nonynyl group, 4-methyl-6-nonynyl group, 2,4-dimethyl-6-nonynyl group, 4,4-dimethyl-6-nonynyl group, 2,4,4-trimethyl-6-nonynyl group, 5-methyl-6-nonynyl group, 2,5-dimethyl-6-nonynyl group, 8-methyl-6-nonynyl group, 2,8-dimethyl-6-nonynyl group, 8,8-dimethyl-6-nonynyl group, 2,8,8-trimethyl-6-nonynyl group, 3-methyl-5-nonynyl group, 2,3-dimethyl-5-nonynyl group, 3,3-dimethyl-5-nonynyl group, 2,3,3-trimethyl-5-nonynyl group, 4-methyl-5-nonynyl group, 2,4-dimethyl-5-nonynyl group, 4,4-dimethyl-5-nonynyl group, 2,4,4-trimethyl-5-nonynyl group, 7-methyl-5-nonynyl group, 2,7-dimethyl-5-nonynyl group, 7-ethyl-5-nonynyl group, 7-ethyl-2-methyl-5-nonynyl group, 8-methyl-5-nonynyl group, 2,8-dimethyl-5-nonynyl group, 8,8-dimethyl-5-nonynyl group, 2,8,8-trimethyl-5-nonynyl group, 3-methyl-4-nonynyl group, 2,3-dimethyl-4-nonynyl group, 6-methyl-4-nonynyl group, 2,6-dimethyl-4-nonynyl group, 6, 6-dimethyl-4-nonynyl group, 2,6,6-trimethyl-4-nonynyl group, 7,7-dimethyl-4-nonynyl group, 2,7,7-trimethyl-4-nonynyl group, 7-ethyl-4-nonynyl group, 7-ethyl-2-methyl-4-nonynyl group, etc. may be mentioned. Among these, a $C_4$ to $C_{10}$ alkynyl group, in particular a $C_5$ to $C_{10}$ alkynyl group is preferred.

As the substituent of these $R^{21}$, fluorine, chlorine, and other halogen atoms; the methoxy group, ethoxy group, propoxy group, butoxy group, and other lower alkoxy groups; the cyclopentyl group, cyclohexyl group, and other $C_3$ to $C_8$ cycloalkyl groups; etc. may be mentioned. Among these, the lower alkoxy groups and cycloalkyl groups are preferred.

In the $R^{21}$ in the $R^2$ of the above said formula (I), $R^{21}$ is preferably a $C_3$ to $C_7$ straight or branched alkyl group. As the $C_3$ to $C_7$ alkyl group, for example, the n-propyl group, n-butyl group, n-pentyl group, n-hexyl group, n-heptyl group, 2-methylhexyl group, 2-methylpentyl group, etc. may be mentioned.

Further, the $R^{22}$ when the $R^2$ relating to the above formula (I) is $(CH_2)_nR^{22}$ is a $C_3$ to $C_{10}$ cycloalkyl group which may be substituted. As specific examples of the cycloalkyl group in this case, the cyclopentyl group, cyclohexyl group, etc. may be mentioned. Among these, a $C_3$ to $C_8$ cycloalkyl group, in particular a $C_4$ to $C_7$ cycloalkyl group, is preferred. Note that as the substituent, for example, a methyl group, ethyl group, propyl group, hexyl group, or other $C_1$ to $C_6$ lower alkyl group; a fluorine, chlorine, or other halogen atom; the methoxy group, ethoxy group, propoxy group, butoxy group, and other lower alkoxy groups; the trifluoromethyl group and other halogenated lower alkyl groups, etc. may be mentioned. Further, n is an integer of 0 to 2.

In the above formula (I), $R^3$ and $R^4$ are each independent, that is, may be the same or different, and represent a hydrogen atom or formula (II):

$$-\underset{\underset{O}{\|}}{C}-R^5 \qquad (II)$$

where, $R^5$ is a $C_1$ to $C_5$ straight or branched alkyl group, as specific examples of which the same examples as of $R^2$ may be mentioned. As the $R^5$, a $C_1$ to $C_3$ alkyl group is preferable, in particular, a straight $C_1$ - $C_3$, alkyl group is preferable. Note that as $R^5$, in particular a methyl group is preferred. Further, as $R^3$ and $R^4$, in particular a hydrogen atom is preferred.

The configurations of the 8-position, 9-position, 11-position, 12-position, and 15-position of the prostacyclin of the above said formula (I) are the same as with natural prostacyclin and this in particular is a useful stereo isomer, but the present invention includes stereo isomers with differing configurations at those positions and mixtures of any proportions thereof.

Preferable examples of the prostacyclins which may be used in the present invention are as follows.

(1) 9(O)-methano-$\Delta^{6(9\alpha)}$-prostaglandin $I_1$
(2) 20-methyl-9(O)-methano-$\Delta^{6(9\alpha)}$-prostaglandin $I_1$
(3) 19,20-dinor-9(O)-methano-$\Delta^{6(9\alpha)}$-prostaglandin $I_1$
(4) 20-nor-9(O)-methano-$\Delta^{6(9\alpha)}$-prostaglandin $I_1$
(5) 19-methyl-9(O)-methano-$\Delta^{6(9\alpha)}$-prostaglandin $I_1$
(6) 18-methyl-9(O)-methano-$\Delta^{6(9\alpha)}$-prostaglandin $I_1$

(7) 20-ethyl-9(O)-methano-$\Delta^{6(9\alpha)}$-prostaglandin $I_1$
(8) 20-isopropyl-9(O)-methano-$\Delta^{6(9\alpha)}$-prostaglandin $I_1$
(9) 20-methyl-20-ethyl-9(O)-methano-$\Delta^{6(9\alpha)}$-prostaglandin $I_1$
(10) 19-methyl-20-ethyl-9(O)-methano-$\Delta^{6(9\alpha)}$-prostaglandin $I_1$
(11) 18-methyl-20-ethyl-9(O)-methano-$\Delta^{6(9\alpha)}$-prostaglandin $I_1$
(12) 20-propyl-9(O)-methano-$\Delta^{6(9\alpha)}$-prostaglandin $I_1$
(13) 20-(sec-butyl)-9(O)-methano-$\Delta^{6(9\alpha)}$-prostaglandin $I_1$
(14) 20-(2-butyl)-9(O)-methano-$\Delta^{6(9\alpha)}$-prostaglandin $I_1$
(15) 20-methyl-20-propyl-9(O)-methano-$\Delta^{6(9\alpha)}$-prostaglandin $I_1$
(16) 19-methyl-20-propyl-9(O)-methano-$\Delta^{6(9\alpha)}$-prostaglandin $I_1$
(17) 18-methyl-20-propyl-9(O)-methano-$\Delta^{6(9\alpha)}$-prostaglandin $I_1$
(18) 20-nor-18-methylene-9(O)-methano-$\Delta^{6(9\alpha)}$-prostaglandin $I_1$
(19) 20-nor-18,19-didehydro-9(O)-methano-$\Delta^{6(9\alpha)}$-prostaglandin $I_1$
(20) 18,19-didehydro-9(O)-methano-$\Delta^{6(9\alpha)}$-prostaglandin $I_1$
(21) 18,19-didehydro-20-methyl-9(O)-methano-$\Delta^{6(9\alpha)}$-prostaglandin $I_1$
(22) 18,19-didehydro-20-ethyl-9(O)-methano-$\Delta^{6(9\alpha)}$-prostaglandin $I_1$
(23) 18,19-didehydro-20-propyl-9(O)-methano-$\Delta^{6(9\alpha)}$-prostaglandin $I_1$
(24) 18,19-didehydro-20-butyl-9(O)-methano-$\Delta^{6(9\alpha)}$-prostaglandin $I_1$
(25) 18-methylene-20-methyl-9(O)-methano-$\Delta^{6(9\alpha)}$-prostaglandin $I_1$
(26) 18,19-didehydro-20,20-dimethyl-9(O)-methano-$\Delta^{6(9\alpha)}$-prostaglandin $I_1$
(27) 18,19-didehydro-20-methyl-20-ethyl-9(O)-methano-$\Delta^{6(9\alpha)}$-prostaglandin $I_1$
(28) 18,19-didehydro-20-isopropyl-20-ethyl-9(O)-methano-$\Delta^{6(9\alpha)}$-prostaglandin $I_1$
(29) 18,19-didehydro-20-methyl-20-butyl-9(O)-methano-$\Delta^{6(9\alpha)}$-prostaglandin $I_1$
(30) 18,19-didehydro-20-(1-methylpropyl)-9(O)-methano-$\Delta^{6(9\alpha)}$-prostaglandin $I_1$
(31) 18,19-didehydro-20-methyl-20-propyl-9(O)-methano-$\Delta^{6(9\alpha)}$-prostaglandin $I_1$
(32) 18,19-didehydro-20,20-dimethyl-20-butyl-9(O)-methano-$\Delta^{6(9\alpha)}$-prostaglandin $I_1$
(33) 19,20-didehydro-9(O)-methano-$\Delta^{6(9\alpha)}$-prostaglandin $I_1$
(34) 19,20-didehydro-20-methyl-9(O)-methano-$\Delta^{6(9\alpha)}$-prostaglandin $I_1$
(35) 19,20-didehydro-19-methyl-9(O)-methano-$\Delta^{6(9\alpha)}$-prostaglandin $I_1$
(36) 19,20-didehydro-20-ethyl-9(O)-methano-$\Delta^{6(9\alpha)}$-prostaglandin $I_1$
(37) 19,20-didehydro-20-propyl-9(O)-methano-$\Delta^{6(9\alpha)}$-prostaglandin $I_1$
(38) 19,20-didehydro-20-butyl-9(O)-methano-$\Delta^{6(9\alpha)}$-prostaglandin $I_1$
(39) 19,20-didehydro-18,20-dimethyl-9(O)-methano-$\Delta^{6(9\alpha)}$-prostaglandin $I_1$
(40) 19,20-didehydro-20-(1-methylpropyl)-9(O)-methano-$\Delta^{6(9\alpha)}$-prostaglandin $I_1$
(41) 19,20-didehydro-20-isopropyl-9(O)-methano-$\Delta^{6(9\alpha)}$-prostaglandin $I_1$
(42) 19,20-didehydro-20-(2-methylbutyl)-9(O)-methano-$\Delta^{6(9\alpha)}$-prostaglandin $I_1$
(43) 19,20-didehydro-20-(1,1-dimethylpropyl)-9(O)-methano-$\Delta^{6(9\alpha)}$-prostaglandin $I_1$
(44) 20-methylene-9(O)-methano-$\Delta^{6(9\alpha)}$-prostaglandin $I_1$
(45) 20-methyl-20-methylene-9(O)-methano-$\Delta^{6(9\alpha)}$-prostaglandin $I_1$
(46) 19-methyl-20-methylene-9(O)-methano-$\Delta^{6(9\alpha)}$-prostaglandin $I_1$
(47) 20-ethylidene-9(O)-methano-$\Delta^{6(9\alpha)}$-prostaglandin $I_1$
(48) 20-propylidene-9(O)-methano-$\Delta^{6(9\alpha)}$-prostaglandin $I_1$
(49) 20-butylidene-9(O)-methano-$\Delta^{6(9\alpha)}$-prostaglandin $I_1$
(50) 20-(1-ethylbutylidene)-9(O)-methano-$\Delta^{6(9\alpha)}$-prostaglandin $I_1$
(51) 20-(1-methylbutylidene)-9(O)-methano-$\Delta^{6(9\alpha)}$-prostaglandin $I_1$
(52) 20-(1-methylethylidene)-9(O)-methano-$\Delta^{6(9\alpha)}$-prostaglandin $I_1$
(53) 19-methyl-20-propylidene-9(O)-methano-$\Delta^{6(9\alpha)}$-prostaglandin $I_1$
(54) 20-(2-methylpropyiidene)-9(O)-methano-$\Delta^{6(9\alpha)}$-prostaglandin $I_1$
(55) 19-methyl-20-butylidene-9(O)-methano-$\Delta^{6(9\alpha)}$-prostaglandin $I_1$
(56) 20-(3-methylbutylidene)-9(O)-methano-$\Delta^{6(9\alpha)}$-prostaglandin $I_1$
(57) 19,19-dimethyl-20-methylene-9(O)-methano-$\Delta^{6(9\alpha)}$-prostaglandin $I_1$
(58) 20-vinyl-9(O)-methano-$\Delta^{6(9\alpha)}$-prostaglandin $I_1$
(59) 19-methyl-20-vinyl-9(O)-methano-$\Delta^{6(9\alpha)}$-prostaglandin $I_1$
(60) 19,19-dimethyl-20-vinyl-9(O)-methano-$\Delta^{6(9\alpha)}$-prostaglandin $I_1$
(61) 20-methyl-20-vinyl-9(O)-methano-$\Delta^{6(9\alpha)}$-prostaglandin $I_1$
(62) 20-(1-methylvinyl)-9(O)-methano-$\Delta^{6(9\alpha)}$-prostaglandin $I_1$
(63) 20-(1-propenyl-)-9(O)-methano-$\Delta^{6(9\alpha)}$-prostaglandin $I_1$
(64) 20-(2-methyl-1-propenyl)-9(O)-methano-$\Delta^{6(9\alpha)}$-prostaglandin $I_1$
(65) 19-methyl-20-(1-propenyl)-9(O)-methano-$\Delta^{6(9\alpha)}$-prostaglandin $I_1$

(66) 19,19-dimethyl-20-(1-propenyl)-9(O)-methano-$\Delta^{6(9\alpha)}$-prostaglandin $I_1$
(67) 19-methyl-20-(2-methyl-1-propenyl)-9(O)-methano-$\Delta^{6(9\alpha)}$-prostaglandin $I_1$
(68) 18-methyl-20-(2-methyl-1-propenyl)-9(O)-methano-$\Delta^{6(9\alpha)}$-prostaglandin $I_1$
(69) 18,18-dimethyl-20-(2-methyl-1-propenyl)-9-(O)-methano-$\Delta^{6(9\alpha)}$-prostaglandin $I_1$
(70) 20-(1-butenyl)-9(O)-methano-$\Delta^{6(9\alpha)}$-prostaglandin $I_1$
(71) 20-(2-methyl-1-butenyl)-9(O)-methano-$\Delta^{6(9\alpha)}$-prostaglandin $I_1$
(72) 20-(2-ethyl-1-butenyl)-9(O)-methano-$\Delta^{6(9\alpha)}$-prostaglandin $I_1$
(73) 20-methyl-20-(1-butenyl)-9(O)-methano-$\Delta^{6(9\alpha)}$-prostaglandin $I_1$
(74) 19-methyl-20-(1-butenyl)-9(O)-methano-$\Delta^{6(9\alpha)}$-prostaglandin $I_1$
(75) 19,19-dimethyl-20-(1-butenyl)-9(O)-methano-$\Delta^{6(9\alpha)}$-prostaglandin $I_1$
(76) 20-nor-18,19-tetrahydro-9(O)-methano-$\Delta^{6(9\alpha)}$-prostaglandin $I_1$
(77) 18,19-tetrahydro-9(O)-methano-$\Delta^{6(9\alpha)}$-prostaglandin $I_1$
(78) 18,19-tetrahydro-20-methyl-9(O)-methano-$\Delta^{6(9\alpha)}$-prostaglandin $I_1$
(79) 18,19-tetrahydro-20-ethyl-9(O)-methano-$\Delta^{6(9\alpha)}$-prostaglandin $I_1$
(80) 18,19-tetrahydro-20-(2-methylbutyl)-9(O)-methano-$\Delta^{6(9\alpha)}$-prostaglandin $I_1$
(81) 18,19-tetrahydro-20-propyl-9(O)-methano-$\Delta^{6(9\alpha)}$-prostaglandin $I_1$
(82) 18,19-tetrahydro-20,20-dimethyl-9(O)-methano-$\Delta^{6(9\alpha)}$-prostaglandin $I_1$
(83) 18,19-tetrahydro-20,20,20-trimethyl-9(O)-methano-$\Delta^{6(9\alpha)}$-prostaglandin $I_1$
(84) 18,19-tetrahydro-20-methyl-20-ethyl-9(O)-methano-$\Delta^{6(9\alpha)}$-prostaglandin $I_1$
(85) 18,19-tetrahydro-20,20-dimethyl-20-ethyl-9(O)-methano-$\Delta^{6(9\alpha)}$-prostaglandin $I_1$
(86) 18,19-tetrahydro-20,20-diethyl-9(O)-methano-$\Delta^{6(9\alpha)}$-prostaglandin $I_1$
(87) 18,19-tetrahydro-20-isopropyl-9(O)-methano-$\Delta^{6(9\alpha)}$-prostaglandin $I_1$
(88) 18,19-tetrahydro-20-t-butyl-9(O)-methano-$\Delta^{6(9\alpha)}$-prostaglandin $I_1$
(89) 18,19-tetrahydro-20-methyl-20-propyl-9(O)-methano-$\Delta^{6(9\alpha)}$-prostaglandin $I_1$
(90) 18,19-tetrahydro-20,20-dimethyl-20-propyl-9(O)-methano-$\Delta^{6(9\alpha)}$-prostaglandin $I_1$
(91) 18,19-tetrahydro-20-(2-methylpropyl)-9(O)-methano-$\Delta^{6(9\alpha)}$-prostaglandin $I_1$
(92) 18,19-tetrahydro-20-(2,2-dimethylpropyl)-9(O)-methano-$\Delta^{6(9\alpha)}$-prostaglandin $I_1$
(93) 18,19-tetrahydro-20-methyl-20-butyl-9(O)-methano-$\Delta^{6(9\alpha)}$-prostaglandin $I_1$
(94) 17,20-dimethyl-9(O)-methano-$\Delta^{6(9\alpha)}$-prostaglandin $I_1$
(95) 18,19-tetrahydro-20-(2-methylbutyl)-9(O)-methano-$\Delta^{6(9\alpha)}$-prostaglandin $I_1$
(96) 18,19-tetrahydro-20-(2-ethylbutyl)-9(O)-methano-$\Delta^{6(9\alpha)}$-prostaglandin $I_1$
(97) 18,19-tetrahydro-20-(2,2-dimethylbutyl)-9(O)-methano-$\Delta^{6(9\alpha)}$-prostaglandin $I_1$
(98) 19,20-tetrahydro-9(O)-methano-$\Delta^{6(9\alpha)}$-prostaglandin $I_1$
(99) 19,20-tetrahydro-18-methyl-9(O)-methano-$\Delta^{6(9\alpha)}$-prostaglandin $I_1$
(100) 19,20-tetrahydro-18,20-dimethyl-9(O)-methano-$\Delta^{6(9\alpha)}$-prostaglandin $I_1$
(101) 19,20-tetrahydro-20-methyl-9(O)-methano-$\Delta^{6(9\alpha)}$-prostaglandin $I_1$
(102) 19,20-tetrahydro-20-ethyl-9(O)-methano-$\Delta^{6(9\alpha)}$-prostaglandin $I_1$
(103) 19,20-tetrahydro-20-isopropyl-9(O)-methano-$\Delta^{6(9\alpha)}$-prostaglandin $I_1$
(104) 19,20-tetrahydro-20-t-butyl-9(O)-methano-$\Delta^{6(9\alpha)}$-prostaglandin $I_1$
(105) 19,20-tetrahydro-20-propyl-9(O)-methano-$\Delta^{6(9\alpha)}$-prostaglandin $I_1$
(106) 19,20-tetrahydro-20-(1-methylpropyl)-9(O)-methano-$\Delta^{6(9\alpha)}$-prostaglandin $I_1$
(107) 19,20-tetrahydro-20-(1-ethylpropyl)-9(O)-methano-$\Delta^{6(9\alpha)}$-prostaglandin $I_1$
(108) 19, 20-tetrahydro-20-(1,1-dimethylpropyl)-9(O)-methano-$\Delta^{6(9\alpha)}$-prostaglandin $I_1$
(109) 19,20-tetrahydro-20-butyl-9(O)-methano-$\Delta^{6(9\alpha)}$-prostaglandin $I_1$
(110) 19,20-tetrahydro-18,18-dimethyl-20-butyl-9(O)-methano-$\Delta^{6(9\alpha)}$-prostaglandin $I_1$
(111) 19,20-tetrahydro-20-(1-methylbutyl)-9(O)-methano-$\Delta^{6(9\alpha)}$-prostaglandin $I_1$
(112) 19,20-tetrahydro-20-(1,1-dimethylbutyl)-9(O)-methano-$\Delta^{6(9\alpha)}$-prostaglandin $I_1$
(113) 19, 20-tetrahydro-20-(2,2-dimethylbutyl)-9(O)-methano-$\Delta^{6(9\alpha)}$-prostaglandin $I_1$
(114) 19,20-tetrahydro-20-(2-ethylbutyl)-9(O)-methano-$\Delta^{6(9\alpha)}$-prostaglandin $I_1$
(115) 19,20-tetrahydro-18-methyl-20-propyl-9(O)-methano-$\Delta^{6(9\alpha)}$-prostaglandin $I_1$
(116) 20-methylidyne-9(O)-methano-$\Delta^{6(9\alpha)}$-prostaglandin $I_1$
(117) 20-methylidyne-18-methyl-9(O)-methano-$\Delta^{6(9\alpha)}$-prostaglandin $I_1$
(118) 20-ethylidyne-9(O)-methano-$\Delta^{6(9\alpha)}$-prostaglandin $I_1$
(119) 20-propylidyne-9(O)-methano-$\Delta^{6(9\alpha)}$-prostaglandin $I_1$
(120) 20-(2-methylpropylidyne)-9(O)-methano-$\Delta^{6(9\alpha)}$-prostaglandin $I_1$
(121) 20-propylidyne-19-methyl-9(O)-methano-$\Delta^{6(9\alpha)}$-prostaglandin $I_1$
(122) 20-(2,2-dimethylpropylidyne)-9(O)-methano-$\Delta^{6(9\alpha)}$-prostaglandin $I_1$
(123) 20-prolidyne-19, 19-dimethyl-9(O)-methano-$\Delta^{6(9\alpha)}$-prostaglandin $I_1$
(124) 20-butylidyne-9(O)-methano-$\Delta^{6(9\alpha)}$-prostaglandin $I_1$

(125) 20-butylidyne-18-methyl-9(O)-methano-$\Delta^{6(9\alpha)}$-prostaglandin $I_1$
(126) 20-butylidyne-18,18-dimethyl-9(O)-methano-$\Delta^{6(9\alpha)}$-prostaglandin $I_1$
(127) 20-butylidyne-19-methyl-9(O)-methano-$\Delta^{6(9\alpha)}$-prostaglandin $I_1$
(128) 20-butylidyne-19,19-dimethyl-9(O)-methano-$\Delta^{6(9\alpha)}$-prostaglandin $I_1$
(129) 20-(2-methylbutylidyne)-9(O)-methano-$\Delta^{6(9\alpha)}$-prostaglandin $I_1$
(130) 20-(2-ethylbutylidyne)-9(O)-methano-$\Delta^{6(9\alpha)}$-prostaglandin $I_1$
(131) 20-(3-methylbutylidyne)-9(O)-methano-$\Delta^{6(9\alpha)}$-prostaglandin $I_1$
(132) 20-(3,3-dimethylbutylidyne)-9(O)-methano-$\Delta^{6(9\alpha)}$-prostaglandin $I_1$
(133) 20-ethynyl-9(O)-methano-$\Delta^{6(9\alpha)}$-prostaglandin $I_1$
(134) 20-ethynyl-18-methyl-9(O)-methano-$\Delta^{6(9\alpha)}$-prostaglandin $I_1$
(135) 20-ethynyl-19-methyl-9(O)-methano-$\Delta^{6(9\alpha)}$-prostaglandin $I_1$
(136) 20-ethynyl-19,19-dimethyl-9(O)-methano-$\Delta^{6(9\alpha)}$-prostaglandin $I_1$
(137) 20-ethynyl-20-methyl-9(O)-methano-$\Delta^{6(9\alpha)}$-prostaglandin $I_1$
(138) 20-ethynyl-20,20-dimethyl-9(O)-methano-$\Delta^{6(9\alpha)}$-prostaglandin $I_1$
(139) 20-(1-propynyl)-9(O)-methano-$\Delta^{6(9\alpha)}$-prostaglandin $I_1$
(140) 20-(1-propynyl)-18-methyl-9(O)-methano-$\Delta^{6(9\alpha)}$-prostaglandin $I_1$
(141) 20-(1-propynyl)-19-methyl-9(O)-methano-$\Delta^{6(9\alpha)}$-prostaglandin $I_1$
(142) 20-(1-propynyl)-19,19-dimethyl-9(O)-methano-$\Delta^{6(9\alpha)}$-prostaglandin $I_1$
(143) 20-(1-propynyl)-20-methyl-9(O)-methano-$\Delta^{6(9\alpha)}$-prostaglandin $I_1$
(144) 20-(1-propynyl)-20,20-dimethyl-9(O)-methano-$\Delta^{6(9\alpha)}$-prostaglandin $I_1$
(145) 20-(1-butynyl)-9(O)-methano-$\Delta^{6(9\alpha)}$-prostaglandin $I_1$
(146) 20-(1-butynyl)-18-methyl-9(O)-methano-$\Delta^{6(9\alpha)}$-prostaglandin $I_1$
(147) 20-(1-butynyl)-19-methyl-9(O)-methano-$\Delta^{6(9\alpha)}$-prostaglandin $I_1$
(148) 20-(1-butynyl)-20-methyl-9(O)-methano-$\Delta^{6(9\alpha)}$-prostaglandin $I_1$
(149) 20-(1-butynyl)-19,19-dimethyl-9(O)-methano-$\Delta^{6(9\alpha)}$-prostaglandin $I_1$
(150) 20-(3-methyl-1-butynyl)-9(O)-methano-$\Delta^{6(9\alpha)}$-prostaglandin $I_1$
(151) 20-(3,3-dimethyl-1-butynyl)-9(O)-methano-$\Delta^{6(9\alpha)}$-prostaglandin $I_1$
(152) 20-(2-butynyl)-9(O)-methano-$\Delta^{6(9\alpha)}$-prostaglandin $I_1$
(153) 20-(2-propynyl)-18-methyl-9(O)-methano-$\Delta^{6(9\alpha)}$-prostaglandin $I_1$
(154) 20-(2-propynyl)-18,18-dimethyl-9(O)-methano-$\Delta^{6(9\alpha)}$-prostaglandin $I_1$
(155) 20-(2-propynyl)-19-methyl-9(O)-methano-$\Delta^{6(9\alpha)}$-prostaglandin $I_1$
(156) 20-(2-propynyl)-19,19-dimethyl-9(O)-methano-$\Delta^{6(9\alpha)}$-prostaglandin $I_1$
(157) 20-(2-propynyl)-20-methyl-9(O)-methano-$\Delta^{6(9\alpha)}$-prostaglandin $I_1$
(158) 20-(1-methyl-2-propynyl)-9(O)-methano-$\Delta^{6(9\alpha)}$-prostaglandin $I_1$
(159) 20-(1,1-dimethyl-2-propynyl)-9(O)-methano-$\Delta^{6(9\alpha)}$-prostaglandin $I_1$
(160) 20-(2-butynyl)-18-methyl-9(O)-methano-$\Delta^{6(9\alpha)}$-prostaglandin $I_1$
(161) 20-(2-butynyl)-18,18-dimethyl-9(O)-methano-$\Delta^{6(9\alpha)}$-prostaglandin $I_1$
(162) 20-(2-butynyl)-19,19-dimethyl-9(O)-methano-$\Delta^{6(9\alpha)}$-prostaglandin $I_1$
(161) 20-(2-butynyl)-20,20-dimethyl-9(O)-methano-$\Delta^{6(9\alpha)}$-prostaglandin $I_1$
(162) 20-(1-methyl-2-butynyl)-9(O)-methano-$\Delta^{6(9\alpha)}$-prostaglandin $I_1$
(163) 20-(1,1-dimethyl-2-butynyl)-9(O)-methano-$\Delta^{6(9\alpha)}$-prostaglandin $I_1$
(164) 20-(3-butynyl)-9(O)-methano-$\Delta^{6(9\alpha)}$-prostaglandin $I_1$
(165) 20-(3-butynyl)-18,18-dimethyl-9(O)-methano-$\Delta^{6(9\alpha)}$-prostaglandin $I_1$
(166) 20-(3-butynyl)-18-methyl-9(O)-methano-$\Delta^{6(9\alpha)}$-prostaglandin $I_1$
(167) 20-(3-butynyl)-20-methyl-9(O)-methano-$\Delta^{6(9\alpha)}$-prostaglandin $I_1$
(168) 20-(2-methyl-3-butynyl)-9(O)-methano-$\Delta^{6(9\alpha)}$-prostaglandin $I_1$
(169) 20-(2,2-dimethyl-3-butynyl)-9(O)-methano-$\Delta^{6(9\alpha)}$-prostaglandin $I_1$
(170) 20-(1,1-dimethyl-3-butynyl)-9(O)-methano-$\Delta^{6(9\alpha)}$-prostaglandin $I_1$
(171) 16,17,18,19,20-pentanor-15-cyclopentyl-9(O)-methano-$\Delta^{6(9\alpha)}$-prostaglandin $I_1$
(172) 16,17,18,19,20-pentanor-15-cyclohexyl-9(O)-methano-$\Delta^{6(9\alpha)}$-prostaglandin $I_1$
(173) 16,17,18,19,20-pentanor-15-(2-chlorocyclopentyl)-9(O)-methano-$\Delta^{6(9\alpha)}$-prostaglandin $I_1$
(174) 16,17,18,19,20-pentanor-15-(3-trifluoromethyl-cyclohexyl)-9(O)-methano-$\Delta^{6(9\alpha)}$-prostaglandin $I_1$
(175) 17-ethoxy-9(O)-methano-$\Delta^{6(9\alpha)}$-prostaglandin $I_1$
(176) 17-methyl-9(O)-methano-$\Delta^{6(9\alpha)}$-prostaglandin $I_1$
(177) 17(R)-methyl-9(O)-methano-$\Delta^{6(9\alpha)}$-prostaglandin $I_1$
(178) 17(S)-methyl-9(O)-methano-$\Delta^{6(9\alpha)}$-prostaglandin $I_1$
(179) 20-nor-17-methyl-9(O)-methano-$\Delta^{6(9\alpha)}$-prostaglandin $I_1$
(180) 17,19-dimethyl-9(O)-methano-$\Delta^{6(9\alpha)}$-prostaglandin $I_1$
(181) 17,18-dimethyl-9(O)-methano-$\Delta^{6(9\alpha)}$-prostaglandin $I_1$

(182) 17-methyl-20-ethyl-9(O)-methano-$\Delta^{6(9\alpha)}$-prostaglandin $I_1$
(183) 17-methyl-20-isopropyl-9(O)-methano-$\Delta^{6(9\alpha)}$-prostaglandin $I_1$
(184) 17,20-dimethyl-20-ethyl-9(O)-methano-$\Delta^{6(9\alpha)}$-prostaglandin $I_1$
(185) 17,19-dimethyl-20-ethyl-9(O)-methano-$\Delta^{6(9\alpha)}$-prostaglandin $I_1$
(186) 17,18-dimethyl-20-ethyl-9(O)-methano-$\Delta^{6(9\alpha)}$-prostaglandin $I_1$
(187) 17-methyl-20-propyl-9(O)-methano-$\Delta^{6(9\alpha)}$-prostaglandin $I_1$
(188) 17-methyl-20-(sec-butyl)-9(O)-methano-$\Delta^{6(9\alpha)}$-prostaglandin $I_1$
(189) 17-methyl-20-(2-butyl)-9(O)-methano-$\Delta^{6(9\alpha)}$-prostaglandin $I_1$
(190) 17,20-dimethyl-20-propyl-9(O)-methano-$\Delta^{6(9\alpha)}$-prostaglandin $I_1$
(191) 17,19-dimethyl-20-propyl-9(O)-methano-$\Delta^{6(9\alpha)}$-prostaglandin $I_1$
(192) 17,18-dimethyl-20-propyl-9(O)-methano-$\Delta^{6(9\alpha)}$-prostaglandin $I_1$
(193) 20-nor-17-methyl-18-methylene-9(O)-methano-$\Delta^{6(9\alpha)}$-prostaglandin $I_1$
(194) 20-nor-18,19-didehydro-17-methyl-9(O)-methano-$\Delta^{6(9\alpha)}$-prostaglandin $I_1$
(195) 18,19-didehydro-17-methyl-9(O)-methano-$\Delta^{6(9\alpha)}$-prostaglandin $I_1$
(196) 18,19-didehydro-17,20-dimethyl-9(O)-methano-$\Delta^{6(9\alpha)}$-prostaglandin $I_1$
(197) 18,19-didehydro-20-ethyl-17-methyl-9(O)-methano-$\Delta^{6(9\alpha)}$-prostaglandin $I_1$
(198) 18,19-didehydro-20-propyl-17-methyl-9(O)-methano-$\Delta^{6(9\alpha)}$-prostaglandin $I_1$
(199) 18,19-didehydro-20-butyl-17-methyl-9(O)-methano-$\Delta^{6(9\alpha)}$-prostaglandin $I_1$
(200) 18-methylene-17,20-dimethyl-9(O)-methano-$\Delta^{6(9\alpha)}$-prostaglandin $I_1$
(201) 18,19-didehydro-17,20,20-trimethyl-9(O)-methano-$\Delta^{6(9\alpha)}$-prostaglandin $I_1$
(202) 18,19-didehydro-17,20-dimethyl-20-ethyl-9(O)-methano-$\Delta^{6(9\alpha)}$-prostaglandin $I_1$
(203) 18,19-didehydro-20-isopropyl-17-methyl-9(O)-methano-$\Delta^{6(9\alpha)}$-prostaglandin $I_1$
(204) 18,19-didehydro-17,20-dimethyl-20-butyl-9(O)-methano-$\Delta^{6(9\alpha)}$-prostaglandin $I_1$
(205) 18,19-didehydro-17-methyl-20-(1-methylpropyl)-9(O)-methano-$\Delta^{6(9\alpha)}$-prostaglandin $I_1$
(206) 18,19-didehydro-17,20-dimethyl-20-propyl-9(O)-methano-$\Delta^{6(9\alpha)}$-prostaglandin $I_1$
(207) 18,19-didehydro-17,20,20-trimethyl-20-butyl-9(O)-methano-$\Delta^{6(9\alpha)}$-prostaglandin $I_1$
(208) 19,20-didehydro-17-methyl-9(O)-methano-$\Delta^{6(9\alpha)}$-prostaglandin $I_1$
(209) 19,20-didehydro-17,20-dimethyl-9(O)-methano-$\Delta^{6(9\alpha)}$-prostaglandin $I_1$
(210) 19,20-didehydro-17,19-dimethyl-9(O)-methano-$\Delta^{6(9\alpha)}$-prostaglandin $I_1$
(211) 19,20-didehydro-17-methyl-20-ethyl-9(O)-methano-$\Delta^{6(9\alpha)}$-prostaglandin $I_1$
(212) 19,20-didehydro-17-methyl-20-propyl-9(O)-methano-$\Delta^{6(9\alpha)}$-prostaglandin $I_1$
(213) 19,20-didehydro-17-methyl-20-butyl-9(O)-methano-$\Delta^{6(9\alpha)}$-prostaglandin $I_1$
(214) 19,20-didehydro-17,18,20-trimethyl-9(O)-methano-$\Delta^{6(9\alpha)}$-prostaglandin $I_1$
(215) 19,20-didehydro-17-methyl-20-(1-methylpropyl)-9(O)-methano-$\Delta^{6(9\alpha)}$-prostaglandin $I_1$
(216) 19,20-didehydro-17-methyl-20-isopropyl-9(O)-methano-$\Delta^{6(9\alpha)}$-prostaglandin $I_1$
(217) 19,20-didehydro-17-methyl-20-(2-methylbutyl)-9(O)-methano-$\Delta^{6(9\alpha)}$-prostaglandin $I_1$
(218) 19,20-didehydro-17-methyl-20-(1,1-dimethylpropyl)-9(O)-methano-$\Delta^{6(9\alpha)}$-prostaglandin $I_1$
(219) 17-methy1-20-methylene-9(O)-methano-$\Delta^{6(9\alpha)}$-prostaglandin $I_1$
(220) 17,20-dimethyl-20-methylene-9(O)-methano-$\Delta^{6(9\alpha)}$-prostaglandin $I_1$
(221) 17,19-dimethyl-20-methylene-9(O)-methano-$\Delta^{6(9\alpha)}$-prostaglandin $I_1$
(222) 17-methyl-20-ethylidene-9(O)-methano-$\Delta^{6(9\alpha)}$-prostaglandin $I_1$
(223) 17-methyl-20-propylidene-9(O)-methano-$\Delta^{6(9\alpha)}$-prostaglandin $I_1$
(224) 17-methyl-20-butylidene-9(O)-methano-$\Delta^{6(9\alpha)}$-prostaglandin $I_1$
(225)17-methyl-20-(1-ethylbutylidene)-9(O)-methano-$\Delta^{6(9\alpha)}$-prostaglandin $I_1$
(226) 17-methyl-20-(1-methylbutylidene)-9(O)-methano-$\Delta^{6(9\alpha)}$-prostaglandin $I_1$
(227) 17-methyl-20-(1-methylethylidene)-9(O)-methano-$\Delta^{6(9\alpha)}$-prostaglandin $I_1$
(228) 17,19-dimethyl-20-propylidene-9(O)-methano-$\Delta^{6(9\alpha)}$-prostaglandin $I_1$
(229) 17-methyl-20-(2-methylpropylidene)-9(O)-methano-$\Delta^{6(9\alpha)}$-prostaglandin $I_1$
(230) 17,19-dimethyl-20-butylidene-9(O)-methano-$\Delta^{6(9\alpha)}$-prostaglandin $I_1$
(231) 17-methyl-20-(3-methylbutylidene)-9(O)-methano-$\Delta^{6(9\alpha)}$-prostaglandin $I_1$
(232) 17,19,19-trimethyl-20-methylene-9(O)-methano-$\Delta^{6(9\alpha)}$-prostaglandin $I_1$
(233) 17-methyl-20-vinyl-9(O)-methano-$\Delta^{6(9\alpha)}$-prostaglandin $I_1$
(234) 17,19-dimethyl-20-vinyl-9(O)-methano-$\Delta^{6(9\alpha)}$-prostaglandin $I_1$
(235) 17,19,19-trimethyl-20-vinyl-9(O)-methano-$\Delta^{6(9\alpha)}$-prostaglandin $I_1$
(236) 17,20-dimethyl-20-vinyl-9(O)-methano-$\Delta^{6(9\alpha)}$-prostaglandin $I_1$
(237) 17-methyl-20-(1-methylvinyl)-9(O)-methano-$\Delta^{6(9\alpha)}$-prostaglandin $I_1$
(238) 17-methyl-20-(1-propenyl)-9(O)-methano-$\Delta^{6(9\alpha)}$-prostaglandin $I_1$
(239) 17-methyl-20-(2-methyl-1-propenyl)-9(O)-methano-$\Delta^{6(9\alpha)}$-prostaglandin $I_1$
(240) 17,19-dimethyl-20-(1-propenyl)-9(O)-methano-$\Delta^{6(9\alpha)}$-prostaglandin $I_1$

(241) 17,19,19-trimethyl-20-(1-propenyl)-9(O)-methano-$\Delta^{6(9\alpha)}$-prostaglandin $I_1$
(242) 17,19-dimethyl-20-(2-methyl-1-propenyl)-9(O)-methano-$\Delta^{6(9\alpha)}$-prostaglandin $I_1$
(243) 17,18-dimethyl-20-(2-methyl-1-propenyl)-9(O)-methano--$\Delta^{6(9\alpha)}$-prostaglandin $I_1$
(244) 17,18,18-trimethyl-20-(2-methyl-1-propenyl)-9(O)-methano-$\Delta^{6(9\alpha)}$-prostaglandin $I_1$
(245) 17-methyl-20-(1-butenyl)-9(O)-methano-$\Delta^{6(9\alpha)}$-prostaglandin $I_1$
(246) 17-methyl-20-(2-methyl-1-butenyl)-9(O)-methano-$\Delta^{6(9\alpha)}$-prostaglandin $I_1$
(247) 17-methyl-20-(2-ethyl-1-butenyl)-9(O)-methano-$\Delta^{6(9\alpha)}$-prostaglandin $I_1$
(248) 17,20-dimethyl-20-butenyl-9(O)-methano-$\Delta^{6(9\alpha)}$-prostaglandin $I_1$
(249) 17,19-dimethyl-20-butenyl-9(O)-methano-$\Delta^{6(9\alpha)}$-prostaglandin $I_1$
(250) 17,19,19-trimethyl-20-butenyl-9(O)-methano-$\Delta^{6(9\alpha)}$-prostaglandin $I_1$
(251) 20-nor-18,19-tetradehydro-17-methyl-9(O)-methano-$\Delta^{6(9\alpha)}$-prostaglandin $I_1$
(252) 18,19-tetradehydro-17-methyl-9(O)-methano-$\Delta^{6(9\alpha)}$-prostaglandin $I_1$
(253) 18,19-tetradehydro-17,20-dimethyl-9(O)-methano-$\Delta^{6(9\alpha)}$-prostaglandin $I_1$
(254) 18,19-tetradehydro-17-methyl-20-ethyl-9(O)-methano-$\Delta^{6(9\alpha)}$-prostaglandin $I_1$
(255) 18,19-tetradehydro-17-methyl-20-butyl-9(O)-methano-$\Delta^{6(9\alpha)}$-prostaglandin $I_1$
(256) 18,19-tetradehydro-17-methyl-20-propyl-9(O)-methano-$\Delta^{6(9\alpha)}$-prostaglandin $I_1$
(257) 18,19-tetradehydro-17,20,20-trimethyl-9(O)-methano-$\Delta^{6(9\alpha)}$-prostaglandin $I_1$
(258) 18,19-tetradehydro-17,20,20,20-tetramethyl-9(O)-methano-$\Delta^{6(9\alpha)}$-prostaglandin $I_1$
(259) 18,19-tetradehydro-17,20-dimethyl-20-ethyl-9(O)-methano-$\Delta^{6(9\alpha)}$-prostaglandin $I_1$
(260) 18,19-tetradehydro-17,20,20-trimethyl-20-ethyl-9(O)-methano-$\Delta^{6(9\alpha)}$-prostaglandin $I_1$
(261) 18,19-tetradehydro-17-methyl-20,20-diethyl-9(O)-methano-$\Delta^{6(9\alpha)}$-prostaglandin $I_1$
(262) 18,19-tetradehydro-17-methyl-20-isopropyl-9(O)-methano-$\Delta^{6(9\alpha)}$-prostaglandin $I_1$
(263) 18,19-tetradehydro-17-methyl-20-t-butyl-9(O)-methano-$\Delta^{6(9\alpha)}$-prostaglandin $I_1$
(264) 18,19-tetradehydro-17,20-dimethyl-20-propyl-9(O)-methano-$\Delta^{6(9\alpha)}$-prostaglandin $I_1$
(265) 18,19-tetradehydro-17,20,20-trimethyl-20-propyl-9(O)-methano-$\Delta^{6(9\alpha)}$-prostaglandin $I_1$
(266) 18,19-tetradehydro-17-methyl-20-(2-methylpropyl)-9(O)-methano-$\Delta^{6(9\alpha)}$-prostaglandin $I_1$
(267) 18,19-tetradehydro-17-methyl-20-(2,2-dimethylpropyl)-9(O)-methano-$\Delta^{6(9\alpha)}$-prostaglandin $I_1$
(268) 18,19-tetradehydro-17,20-dimethyl-20-butyl-9(O)-methano-$\Delta^{6(9\alpha)}$-prostaglandin $I_1$
(269) 18,19-tetradehydro-17,20,20-trimethyl-20-butyl-9(O)-methano-$\Delta^{6(9\alpha)}$-prostaglandin $I_1$
(270) 18,19-tetradehydro-17-methyl-20-(2-methylbutyl)-9(O)-methano-$\Delta^{6(9\alpha)}$-prostaglandin $I_1$
(271) 18,19-tetradehydro-17-methyl-20-(2-ethylbutyl)-9(O)-methano-$\Delta^{6(9\alpha)}$-prostaglandin $I_1$
(272) 18,19-tetradehydro-17-methyl-20-(2,2-dimethylbutyl)-9(O)-methano-$\Delta^{6(9\alpha)}$-prostaglandin $I_1$
(273) 19,20-tetradehydro-17-methyl-9(O)-methano-$\Delta^{6(9\alpha)}$-prostaglandin $I_1$
(274) 19,20-tetradehydro-17,18-dimethyl-9(O)-methano-$\Delta^{6(9\alpha)}$-prostaglandin $I_1$
(275) 19,20-tetradehydro-17,18,20-trimethyl-9(O)-methano-$\Delta^{6(9\alpha)}$-prostaglandin $I_1$
(276) 19,20-tetradehydro-17,20-dimethyl-9(O)-methano-$\Delta^{6(9\alpha)}$-prostaglandin $I_1$
(277) 19,20-tetradehydro-17-methyl-20-ethyl-9(O)-methano-$\Delta^{6(9\alpha)}$-prostaglandin $I_1$
(278) 19,20-tetradehydro-17-methyl-20-isopropyl-9(O)-methano-$\Delta^{6(9\alpha)}$-prostaglandin $I_1$
(279) 19,20-tetradehydro-17-methyl-20-t-butyl-9(O)-methano-$\Delta^{6(9\alpha)}$-prostaglandin $I_1$
(280) 19,20-tetradehydro-17-methyl-20-propyl-9(O)-methano-$\Delta^{6(9\alpha)}$-prostaglandin $I_1$
(281) 19,20-tetradehydro-17-methyl-20-(1-methylpropyl)-9(O)-methano-$\Delta^{6(9\alpha)}$-prostaglandin $I_1$
(282) 19,20-tetradehydro-17-methyl-20-(1-ethylpropyl)-9(O)-methano-$\Delta^{6(9\alpha)}$-prostaglandin $I_1$
(283) 19,20-tetradehydro-17-methyl-20-(1,1-dimethylpropyl)-9(O)-methano-$\Delta^{6(9\alpha)}$-prostaglandin $I_1$
(284) 19,20-tetradehydro-17-methyl-20-butyl-9(O)-methano-$\Delta^{6(9\alpha)}$-prostaglandin $I_1$
(285) 19,20-tetradehydro-17,18-dimethyl-20-butyl-9(O)-methano-$\Delta^{6(9\alpha)}$-prostaglandin $I_1$
(286) 19,20-tetradehydro-17-methyl-20-(1-methylbutyl)-9(O)-methano-$\Delta^{6(9\alpha)}$-prostaglandin $I_1$
(287) 19,20-tetradehydro-17-methyl-20-(2,2-dimethylpropyl)-9(O)-methano$\Delta^{6(9\alpha)}$-prostaglandin $I_1$
(288) 19,20-tetradehydro-17-methyl-20-(2-ethylbutyl)-9(O)-methano-$\Delta^{6(9\alpha)}$-prostaglandin $I_1$
(289) 19,20-tetradehydro-17,18-dimethyl-20-propyl-9(O)-methano-$\Delta^{6(9\alpha)}$-prostaglandin $I_1$
(290) 20-methylidyne-17-methyl-9(O)-methano-$\Delta^{6(9\alpha)}$-prostaglandin $I_1$
(291) 20-methylidyne-17,18-dimethyl-9(O)-methano-$\Delta^{6(9\alpha)}$-prostaglandin $I_1$
(292) 20-methylidyne-17,19-dimethyl-9(O)-methano-$\Delta^{6(9\alpha)}$-prostaglandin $I_1$
(293) 20-ethylidyne-17-methyl-9(O)-methano-$\Delta^{6(9\alpha)}$-prostaglandin $I_1$
(294) 20-propylidyne-17-methyl-9(O)-methano-$\Delta^{6(9\alpha)}$-prostaglandin $I_1$
(295) 20-(2-methylpropylidyne)-17-methyl-9(O)-methano-$\Delta^{6(9\alpha)}$-prostaglandin $I_1$
(296) 20-propylidyne-17,19-dimethyl-9(O)-methano-$\Delta^{6(9\alpha)}$-prostaglandin $I_1$
(297) 20-(2,2-dimethylpropylidyne)-17-methyl-9(O)-methano-$\Delta^{6(9\alpha)}$-prostaglandin $I_1$
(298) 20-propylidyne-17,19,19-trimethyl-9(O)-methano-$\Delta^{6(9\alpha)}$-prostaglandin $I_1$
(299) 20-butylidyne-17-methyl-9(O)-methano-$\Delta^{6(9\alpha)}$-prostaglandin $I_1$

(300) 20-butylidyne-17,18-dimethyl-9(O)-methano-$\Delta^{6(9\alpha)}$-prostaglandin $I_1$
(301) 20-butylidyne-17,18,18-trimethyl-9(O)-methano-$\Delta^{6(9\alpha)}$-prostaglandin $I_1$
(302) 20-butylidyne-17,18,19-trimethyl-9(O)-methano-$\Delta^{6(9\alpha)}$-prostaglandin $I_1$
(303) 20-butylidyne-17,19,19-trimethyl-9(O)-methano-$\Delta^{6(9\alpha)}$-prostaglandin $I_1$
(304) 20-butylidyne-17,19-dimethyl-9(O)-methano-$\Delta^{6(9\alpha)}$-prostaglandin $I_1$
(305) 20-(2-methylbutylidyne)-17-methyl-9(O)-methano-$\Delta^{6(9\alpha)}$-prostaglandin $I_1$
(306) 20-(2-ethylbutylidyne)-17-methyl-9(O)-methano-$\Delta^{6(9\alpha)}$-prostaglandin $I_1$
(307) 20-(3-methylbutylidyne)-17-methyl-9(O)-methano-$\Delta^{6(9\alpha)}$-prostaglandin $I_1$
(308) 20-(3,3-dimethylbutylidyne)-17-methyl-9(O)-methano-$\Delta^{6(9\alpha)}$-prostaglandin $I_1$
(309) 20-ethynyl-17-methyl-9(O)-methano-$\Delta^{6(9\alpha)}$-prostaglandin $I_1$
(310) 20-ethynyl-17,18-dimethyl-9(O)-methano-$\Delta^{6(9\alpha)}$-prostaglandin $I_1$
(311) 20-ethynyl-17,19-dimethyl-9(O)-methano-$\Delta^{6(9\alpha)}$-prostaglandin $I_1$
(312) 20-ethynyl-17,19,19-trimethyl-9(O)-methano-$\Delta^{6(9\alpha)}$-prostaglandin $I_1$
(313) 20-ethynyl-17,20-dimethyl-9(O)-methano-$\Delta^{6(9\alpha)}$-prostaglandin $I_1$
(314) 20-ethynyl-17,20,20-trimethyl-9(O)-methano-$\Delta^{6(9\alpha)}$-prostaglandin $I_1$
(315) 20-(1-propynyl)-17-methyl-9(O)-methano-$\Delta^{6(9\alpha)}$-prostaglandin $I_1$
(316) 20-(1-propynyl)-17,18-dimethyl-9(O)-methano-$\Delta^{6(9\alpha)}$-prostaglandin $I_1$
(317) 20-(1-propynyl)-17,19-dimethyl-9(O)-methano-$\Delta^{6(9\alpha)}$-prostaglandin $I_1$
(318) 20-(1-propynyl)-17,19,19-trimethyl-9(O)-methano-$\Delta^{6(9\alpha)}$-prostaglandin $I_1$
(319) 20-(1-propynyl)-17,20-dimethyl-9(O)-methano-$\Delta^{6(9\alpha)}$-prostaglandin $I_1$
(320) 20-(1-propynyl)-17,20,20-trimethyl-9(O)-methano-$\Delta^{6(9\alpha)}$-prostaglandin $I_1$
(321) 20-(1-butynyl)-17-methyl-9(O)-methano-$\Delta^{6(9\alpha)}$-prostaglandin $I_1$
(322) 20-(1-butynyl)-17,18-dimethyl-9(O)-methano-$\Delta^{6(9\alpha)}$-prostaglandin $I_1$
(323) 20-(1-butynyl)-17,19-dimethyl-9(O)-methano-$\Delta^{6(9\alpha)}$-prostaglandin $I_1$
(324) 20-(1-butynyl)-17,19,19-trimethyl-9(O)-methano-$\Delta^{6(9\alpha)}$-prostaglandin $I_1$
(325) 20-(1-butynyl)-17,20-dimethyl-9(O)-methano-$\Delta^{6(9\alpha)}$-prostaglandin $I_1$
(326) 20-(3-methyl-1-butynyl)-17-methyl-9(O)-methano-$\Delta^{6(9\alpha)}$-prostaglandin $I_1$
(327) 20-(3,3-dimethyl-1-butynyl)-17-methyl-9(O)-methano-$\Delta^{6(9\alpha)}$-prostaglandin $I_1$
(328) 20-(2-butynyl)-17-methyl-9(O)-methano-$\Delta^{6(9\alpha)}$-prostaglandin $I_1$
(329) 20-(2-propynyl)-17,18-dimethyl-9(O)-methano-$\Delta^{6(9\alpha)}$-prostaglandin $I_1$
(330) 20-(2-propynyl)-17,18,18-trimethyl-9(O)-methano-$\Delta^{6(9\alpha)}$-prostaglandin $I_1$
(331) 20-(2-propynyl)-17,19-dimethyl-9(O)-methano-$\Delta^{6(9\alpha)}$-prostaglandin $I_1$
(332) 20-(2-propynyl)-17,19,19-trimethyl-9(O)-methano-$\Delta^{6(9\alpha)}$-prostaglandin $I_1$
(333) 20-(2-propynyl)-17,20-dimethyl-9(O)-methano-$\Delta^{6(9\alpha)}$-prostaglandin $I_1$
(334) 20-(1-methyl-2-propynyl)-17-methyl-9(O)-methano-$\Delta^{6(9\alpha)}$-prostaglandin $I_1$
(335) 20-(1,1-dimethyl-2-propynyl)-17-methyl-9(O)-methano-$\Delta^{6(9\alpha)}$-prostaglandin $I_1$
(336) 20-(2-butynyl)-17-methyl-9(O)-methano-$\Delta^{6(9\alpha)}$-prostaglandin $I_1$
(337) 20-(2-butynyl)-17,18-dimethyl-(O)-methano-$\Delta^{6(9\alpha)}$-prostaglandin $I_1$
(338) 20-(2-butynyl)-17,18,18-trimethyl-9(O)-methano-$\Delta^{6(9\alpha)}$-prostaglandin $I_1$
(339) 20-(2-butynyl)-17,19,19-trimethyl-9(O)-methano-$\Delta^{6(9\alpha)}$-prostaglandin $I_1$
(340) 20-(2-butynyl)-17,20,20-trimethyl-9(O)-methano-$\Delta^{6(9\alpha)}$-prostaglandin $I_1$
(341) 20-(1-methyl-2-butynyl)-17-methyl-9(O)-methano-$\Delta^{6(9\alpha)}$-prostaglandin $I_1$
(342) 20-(1,1-dimethyl-2-butynyl)-17-methyl-9(O)-methano-$\Delta^{6(9\alpha)}$-prostaglandin $I_1$
(343) 20-(3-butynyl)-17-methyl-9(O)-methano-$\Delta^{6(9\alpha)}$-prostaglandin $I_1$
(344) 20-(3-butynyl)-17,18-dimethyl-9(O)-methano-$\Delta^{6(9\alpha)}$-prostaglandin $I_1$
(345) 20-(3-butynyl)-17,18,18-trimethyl-9(O)-methano-$\Delta^{6(9\alpha)}$-prostaglandin $I_1$
(346) 20-(3-butynyl)-17,20-dimethyl-9(O)-methano-$\Delta^{6(9\alpha)}$-prostaglandin $I_1$
(347) 20-(1-methyl-3-butynyl)-17-methyl-9(O)-methano-$\Delta^{6(9\alpha)}$-prostaglandin $I_1$
(348) 20-(2-methyl-3-butynyl)-17-methyl-9(O)-methano-$\Delta^{6(9\alpha)}$-prostaglandin $I_1$
(349) 20-(1,1-dimethyl-3-butynyl)-17-methyl-9(O)-methano-$\Delta^{6(9\alpha)}$-prostaglandin $I_1$
(350) 20-(2,2-dimethyl-3-butynyl)-17-methyl-9(O)-methano-$\Delta^{6(9\alpha)}$-prostaglandin $I_1$
(351) methyl esters of compounds (1) to (350)
(352) ethyl esters of compounds (1) to (350)
(353) butyl esters of compounds (1) to (350)
(354) compounds (94) and (176) to (350) where at the 17-position methyl group is replaced with an ethyl group, however, the present invention is not limited to the above compounds.

The prostacyclins of the above formula (I) are easily produced by known methods. Methods of production are, for example, described in detail in Japanese Unexamined Patent Publication (Kokai) Nos. 59-210044 and 61-197518.

The concentration of the prostacyclin, based upon the total weight of the ointment, may be any concentration so long as it has a therapeutic effect, but for example a range of 0.000001% by weight to 0.1% by weight may be mentioned. As a preferable range, 0.00001% by weight to 0.01% by weight may be mentioned.

As the vaseline usable in the composition of the present invention, which are composed of the main medicinal agents, vaselines, paraffins, and fatty acid esters, white vaseline and yellow vaseline may be mentioned, but white vaseline is preferable. The paraffin in the present invention means paraffin or paraffin wax. The white vaseline is preferably contained in an amount of 80 to 100% by weight. The paraffin is preferably contained in a range of 1 to 25% by weight of the total weight. In particular, a range of 3 to 15% by weight, where a conspicuous improvement of the duration of the medicinal effect is observed is preferred. Further, considering the consistency, a range of 3 to 10% by weight is the most preferred.

Examples of the fatty acid ester are diisopropyl adipate, isopropyl palmitate, and isopropyl myristate. These are solubilizing agents for the active ingredient and also work with the paraffin to control the transdermal absorption. These fatty acid esters may be added in a range of less than 1% by weight of the total weight of the ointment, preferably a range less than 0.5% by weight.

In the composition of the present invention comprising the main medicinal agent, vaselines, and glycols, the vaseline used is preferably white vaseline. For the purpose of adjusting the consistency of the ointment, it is possible to add paraffin and/or liquid paraffin. A glycol performs the role of controlling the release of the active ingredient from the ointment base. Examples are ethylene glycol, propylene glycol, 1,2-butanediol, 1,3-butanediol, 1,4-butanediol, 2,3-butanediol, 1,5-pentanediol, 2,4-pentanediol, 1,6-hexanediol, 1,7-heptanediol, 1,2-octanediol, 1,8-octanediol, 1,9-nonanediol, 1,10-decanediol, pinacol, cyclopentane-1,2-diol, cyclohexane-1,2-diol, cyclohexane-1,4-diol, polyethylene glycol 200, 300, 400, 600, etc.

Among these, propylene glycol, 1,3-butanediol and polyethylene glycol 200, 300, and 400 are preferable.

From the viewpoint of the duration of the medicinal effect, the amount of the glycol added is preferably a range of 0.01 to 10% by weight of the total weight, particularly preferably a range of 0.1 to 5% by weight where a conspicuous prolongation of the duration of the medicinal effect is observed.

Further, in addition to the above basic agent, effective ingredient, transdermal absorption adjuster, and solubilizing agent, optionally, it is possible to suitably add, to an extent not impairing the effect of the present invention, a p-oxybenzoic acid ester, benzoic acid, sodium benzoate, salicylic acid, sorbic acid, potassium sorbate, boric acid, and other preservatives; butylhydroxyanisole, butylhydroxytoluene, and other antioxidants; surfactants; moisture retainers; thickeners; perfume; colors; etc.

The ointment may be produced by ordinary methods. For example, the method where the prostacyclin is added to a solubilizing agent to be dissolved, the resultant solution is added to a mixture of the separately heated and melted ointment active ingredient, transdermal absorption promotor, and other additives, followed by uniformly mixing and cooling.

The above-mentioned prostacyclin-containing composition may be used as an agent for treatment of skin sores, for example, bed sores, burn sores, angiopathic sores, diabetic sores, sores accompanying peripheral circulatory disorders and collagen disorders, and other sores and tremors, Bargar's disease, and Raynaud's disease, and other peripheral circulatory disorders. Further, the prostacyclin-containing composition of the present invention does not exhibit any toxicity which in particular becomes a problem.

The ointment preparation of the present invention not only causes an excellent increase in the blood flow of the skin in the test of the blood flow of the skin mentioned later, but also exhibits a clear prolongation of the duration of the medicinal effect. This is supported by the fact that the active ingredient prostacyclin is quickly and sustainedly absorbed from the ointment preparation of the present invention into the location of the skin to which it was applied and exhibits a medicinal effect over a long period. Nothing beats this in medicinal formulations.

## EXAMPLES

The present invention will now be explained in further detail based upon Examples and Comparative Examples, but the present invention is of course not limited thereto.

### Example 1

4 mg of 9(O)-methano-$\Delta^{6(9\alpha)}$-prostaglandin $I_1$ methyl ester was dissolved in 100g of propylene glycol. 5g of this solution was adjusted to a total weight of 100g by adding it to 95g of molten white vaseline, then the resultant mixture was homogeneously mixed and then cooled to obtain an ointment.

Example 2

5 mg of 17(S),20-dimethyl-9(O)-methano-$\Delta^{6(9\alpha)}$-prostaglandin $I_1$ methyl ester was dissolved in 100g of propylene glycol. 5g of this solution was added to 95g of melted white vaseline to adjust the weight to 100g, then the resultant mixture was homogeneously mixed and then cooled to obtain an ointment.

Example 3

The same method was followed to obtain an ointment, except that ethylene glycol was used instead of the propylene glycol of Example 1.

Example 4

The same method was followed to obtain an ointment, except that 1,3-butylene glycol was used instead of the propylene glycol of Example 1.

Example 5

The same method was followed to obtain an ointment, except that diethylene glycol was used instead of the propylene glycol of Example 1.

Example 6

The same method was followed to obtain an ointment, except that triethylene glycol was used instead of the propylene glycol of Example 1.

Example 7

The same method was followed to obtain an ointment, except that 1,6-hexadiol was used instead of the propylene glycol of Example 1.

Example 8

The same method was followed to obtain an ointment, except that polyethylene glycol was used instead of the propylene glycol of Example 1.

Example 9

800 mg of 9(O)-methano-$\Delta^{6(9\alpha)}$-prostaglandin $I_1$ methyl ester was dissolved in 100g of diisopropyl adipate. 0.25g of this solution was added to a mixture of 10g of melted paraffin and a suitable amount of white vaseline to adjust the total weight to 100g, then the resultant mixture was homogeneously mixed and then cooled to obtain an ointment.

Example 10

800 mg of 9(O)-methano-$\Delta^{6(9\alpha)}$-prostaglandin $I_1$ methyl ester was dissolved in 100g of diisopropyl adipate. 0.25g of the solution was added to a mixture of 5g of melted paraffin, 5g of liquid paraffin, and a suitable amount of white vaseline to adjust the total weight to 100g, then the resultant mixture was homogeneously mixed and then cooled to obtain an ointment.

Example 11

100 mg of 17(S),20-dimethyl-9(O)-methano-$\Delta^{6(9\alpha)}$-prostaglandin $I_1$ methyl ester was dissolved in 100g of diisopropyl adipate. 0.25g of this solution was added to a mixture of 10g of melted paraffin and a suitable amount of white vaseline to adjust the total weight to 100g, then the resultant mixture was homogeneously mixed and then cooled to obtain an ointment.

Comparative Example 1

The same method was followed to obtain an ointment, except that diisopropyl adipate was used instead of the propylene glycol of Example 1.

Comparative Example 2

The same method was followed to obtain an ointment, except that oleic acid was used instead of the propylene glycol of Example 1.

Comparative Example 3

The same method was followed to obtain an ointment, except that isopropyl myristate was used instead of the propylene glycol of Example 1.

Comparative Example 4

The same method was followed to obtain an ointment, except that diisopropyl adipate was used instead of the propylene glycol of Example 2.

Comparative Example 5

The same method was followed to obtain an ointment, except that oleic acid was used instead of the propylene glycol of Example 2.

Comparative Example 6

The same method was followed to obtain an ointment, except that isopropyl myristate was used instead of the propylene glycol of Example 2.

Comparative Example 7

800 mg of 9(O)-methano-$\Delta^{6(9\alpha)}$-prostaglandin $I_1$ methyl ester was dissolved in 100g of diisopropyl adipate. 0.25g of the solution was added to 99.75g of melted white vaseline to adjust the total weight to 100g, then the resultant mixture was homogeneously mixed and then cooled to obtain an ointment.

Comparative Example 8

The same method was followed to obtain an ointment, except that glycerine was used instead of the propylene glycol of Example 1.

Comparative Example 9

The same method was followed to obtain an ointment, except that 1-propanol was used instead of the propylene glycol of Example 1.

Comparative Example 10

The same type of method was followed to obtain an ointment, except that 1-butanol was used instead of the propylene glycol of Example 1.

Comparative Example 11

800 mg of 9(O)-methano-$\Delta^{6(9\alpha)}$-prostaglandin $I_1$ methyl ester was dissolved in 100g of diisopropyl adipate. 0.25g of the solution was added to a mixture of 4.5g of melted liquid paraffin and a suitable amount of white vaseline to adjust the total weight to 100g, then the resultant mixture was homogeneously mixed and then cooled to obtain an ointment.

Comparative Example 12

40 mg of 9(O)-methano-$\Delta^{6(9\alpha)}$-prostaglandin $I_1$ methyl ester was dissolved in 100g of diisopropyl adipate. 5g of the solution was added to a mixture of 10g of melted paraffin and a suitable amount of white vaseline to adjust the total weight to 100g, then the resultant mixture was homogeneously mixed and then cooled to obtain an ointment.

Comparative Example 13

40 mg of 9(O)-methano-$\Delta^{6(9\alpha)}$-prostaglandin $I_1$ methyl ester was dissolved in 100g of diisopropyl adipate. 5g of the solution was added to 95g of melted white vaseline to adjust the total weight to 100g, then the resultant mixture was homogeneously mixed and then cooled to obtain an ointment.

Comparative Example 14

100 mg of 17(S),20-dimethyl-9(O)-methano-$\Delta^{6(9\alpha)}$-prostaglandin $I_1$ methyl ester was dissolved in 100g of diisopropyl adipate. 0.25g of the solution was added to a mixture of 4.5g of melted liquid paraffin and a suitable amount of white vaseline to adjust the total weight to 100g, then the resultant mixture was homogeneously mixed and then cooled to obtain an ointment.

Comparative Example 15

5 mg of 17(S),20-dimethyl-9(O)-methano-$\Delta^{6(9\alpha)}$-prostaglandin $I_1$ methyl ester was dissolved in 100g of diisopropyl adipate. 5g of the solution was added to a mixture of 10g of melted paraffin and a suitable amount of white vaseline to adjust the total weight to 100g, then the resultant mixture was homogeneously mixed and then cooled to obtain an ointment.

Comparative Example 16

5 mg of 17(S),20-dimethyl-9(O)-methano-$\Delta^{6(9\alpha)}$-prostaglandin $I_1$ methyl ester was dissolved in 100g of diisopropyl adipate. 5g of the solution was added to 95g of melted white vaseline to adjust the total weight to 100g, then the resultant mixture was homogeneously mixed and then cooled to obtain an ointment.

Experiment 1: Test on Release of Active Ingredient From Base

To confirm that the ointment of the present invention controls the release of the active ingredient, a test was performed on the release of the active ingredient from the base. 70 mg of the ointments obtained in the Examples and Comparative Examples were uniformly coated on 10 $cm^2$ glass plates which were then immersed in a 40% aqueous solution of ethanol (reservoir solution). Parts of the solutions were taken over time and the amounts of the active ingredient in the solutions were assayed by liquid chromatography.

The amount of the active ingredient released in the reservoir solution as against the amount of the active ingredient in the ointment used was used as the rate of release. The results are shown in Fig. 1, Fig. 2, and Fig. 3. The vertical axis shows the rate of release, while the horizontal axis shows the elapse of time from the application of the composition.

$$\text{Rate of release (\%)} = [(\text{amount of active ingredient in reservoir solution}) / (\text{amount of active ingredient in ointment used})] \times 100$$

From these test results, the ointments according to the Examples of the present invention exhibited a slower release of the active ingredient compared with the ointments of the comparative examples.

Experiment 2: Test of Blood Flow at Location of Skin Applied To

To confirm the efficacy of the ointment of the present invention at the location of the skin applied to, the amount of the blood flowing at the location of administration was measured. Compositions obtained in the Examples and Comparative Examples were coated nonsealingly in 23 mg amounts on 1 cm × 1 cm areas of the thighs of hairless rats anesthetized by urethane.

After coating, a laser doppler blood flow meter was used to measure the flow of blood at the administered locations. The difference before and after coating was used as the increase in blood flow. The results are shown in Fig. 4 and Fig. 5 and Fig. 6 and Fig. 7. The vertical axis shows the difference in the blood flow before and after application as the

increase of the blood flow, while the horizontal axis shows the elapse of time from application of the composition in units of hours.

From these test results, ointments according to the Examples of the present invention are observed to exhibit a pronounced prolongation of the action of increasing the blood flow compared with the ointments of the comparative examples.

INDUSTRIAL APPLICABILITY

The ointment preparation of the present invention exhibited slowing of the release of the active ingredient from the ointment in release tests. Not only was a remarkable increase in the blood flow at the skin observed in tests on the blood flow at the skin, but also a clear prolongation of the medicinal effect was observed. This is fully supported by the fact that ointment preparation of the present invention was quickly absorbed at the location of the skin applied to, the medicinal effect was manifested, and the medicinal effect continued over several hours. Nothing beats this in medicinal formulations.

In this way, the ointment base of the present invention is a superior ointment preparation having desirable effects in applying the prostacyclins of the above formula (I) with their high activity and short effective lives in the body to ulcerous skin diseases and to skin susceptible to ulcerous skin diseases, that is, prolongation of the duration of the medicinal effect, stability of the active ingredient, and low irritation of the skin to which it is applied and therefore can be expected to find use as a medicine for treatment of ulcerous skin diseases, Barger's disease, and other peripheral circulatory disorders.

**Claims**

1. A prostacyclin-containing skin application composition containing a prostacyclin comprising, as a medicinally active component, (i) a prostacyclin having the formula (I):

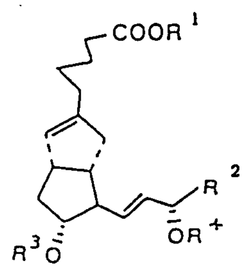

(I)

wherein, $R^1$ represents a hydrogen atom, $C_1$ to $C_{10}$ straight or branched alkyl group, $R^2$ represents $R^{21}$ or $(CH_2)_nR^{22}$ where $R^{21}$ is a $C_3$ to $C_{12}$ or branched alkyl, alkenyl or alkynyl group which may be substituted with, $R^{22}$ represents a $C_3$ to $C_{10}$ cycloalkyl group which may be substituted with, n is an integer of 0 to 2, $R^3$ and $R^4$ are each independently hydrogen atom or formula (II):

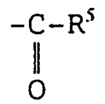

(II)

wherein, $R^5$ represents a $C_1$ to $C_5$ straight or branched alkyl group
and/or its enantiomers, (ii) a vaseline, (iii) a paraffin, and (iv) a fatty acid ester and, optionally, liquid paraffin.

2. A prostacyclin-containing skin application composition as claimed in claim 1, wherein the content of the paraffin is 3 to 10% by weight of the total weight and the content of the fatty acid ester is less than 1% by weight of the total weight.

3. A prostacyclin-containing skin application composition comprising a prostacyclin having the formula (I) and/or its enantiomers, vaseline, and glycol and, optionally, a paraffin and/or liquid paraffin.

4. A prostacyclin-containing skin application composition as claimed in claim 3, wherein the glycol is at least one compound selected from the group consisting of $C_2$ to $C_{10}$ glycols and polyethylene glycols having a melting point of 30°C or less.

5. A prostacyclin-containing skin application composition as claimed in claim 3 or 4, wherein the content of the glycol is 0.1 to 5% by weight of the total weight.

Fig.1

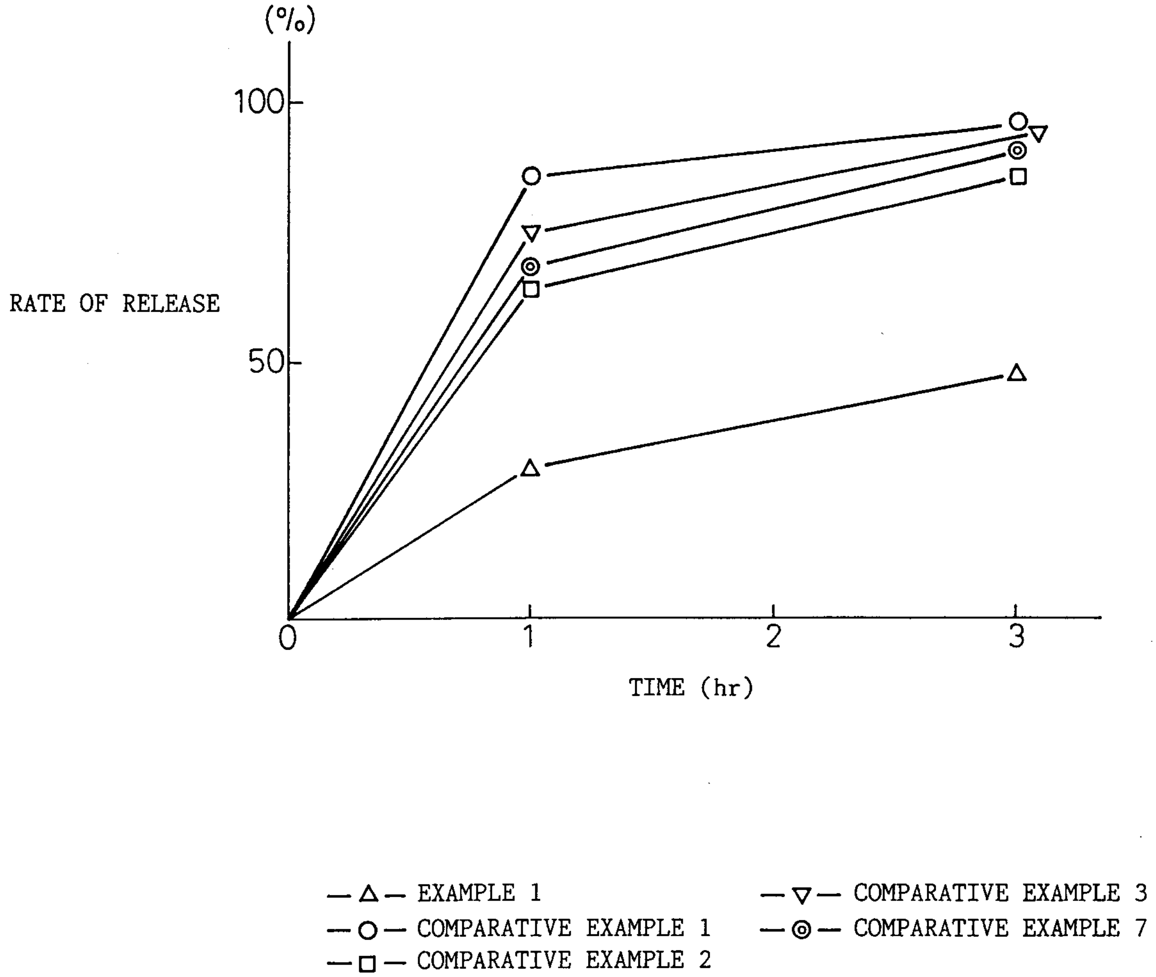
(%)
100
50
RATE OF RELEASE
0
1
2
3
TIME (hr)
—△— EXAMPLE 1
—○— COMPARATIVE EXAMPLE 1
—□— COMPARATIVE EXAMPLE 2
—▽— COMPARATIVE EXAMPLE 3
—◎— COMPARATIVE EXAMPLE 7

Fig.2

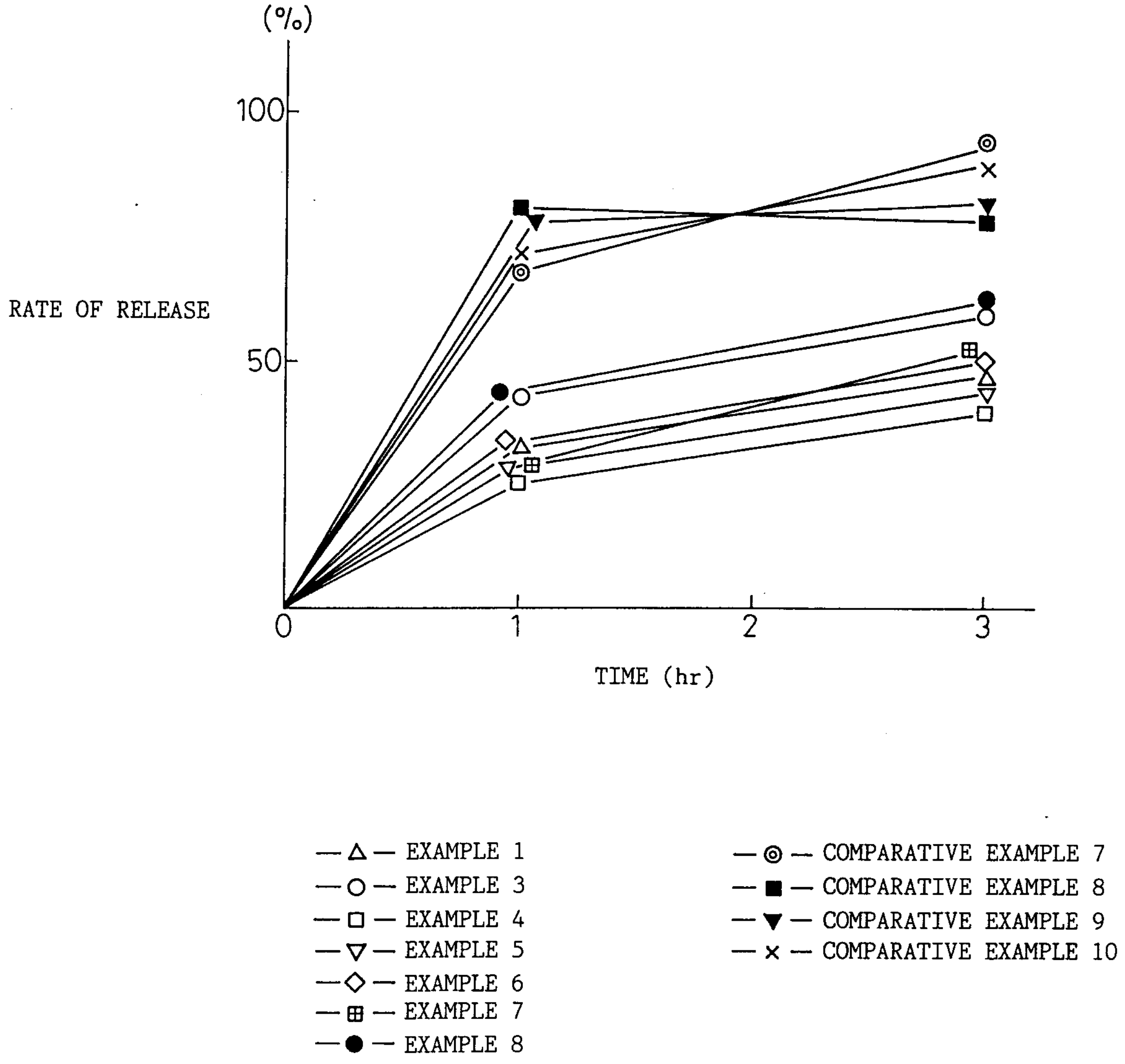
(%)
100
50
RATE OF RELEASE
0
1
2
3
TIME (hr)
—△— EXAMPLE 1
—○— EXAMPLE 3
—□— EXAMPLE 4
—▽— EXAMPLE 5
—◇— EXAMPLE 6
—⊞— EXAMPLE 7
—●— EXAMPLE 8
—◎— COMPARATIVE EXAMPLE 7
—■— COMPARATIVE EXAMPLE 8
—▼— COMPARATIVE EXAMPLE 9
—×— COMPARATIVE EXAMPLE 10

Fig.3

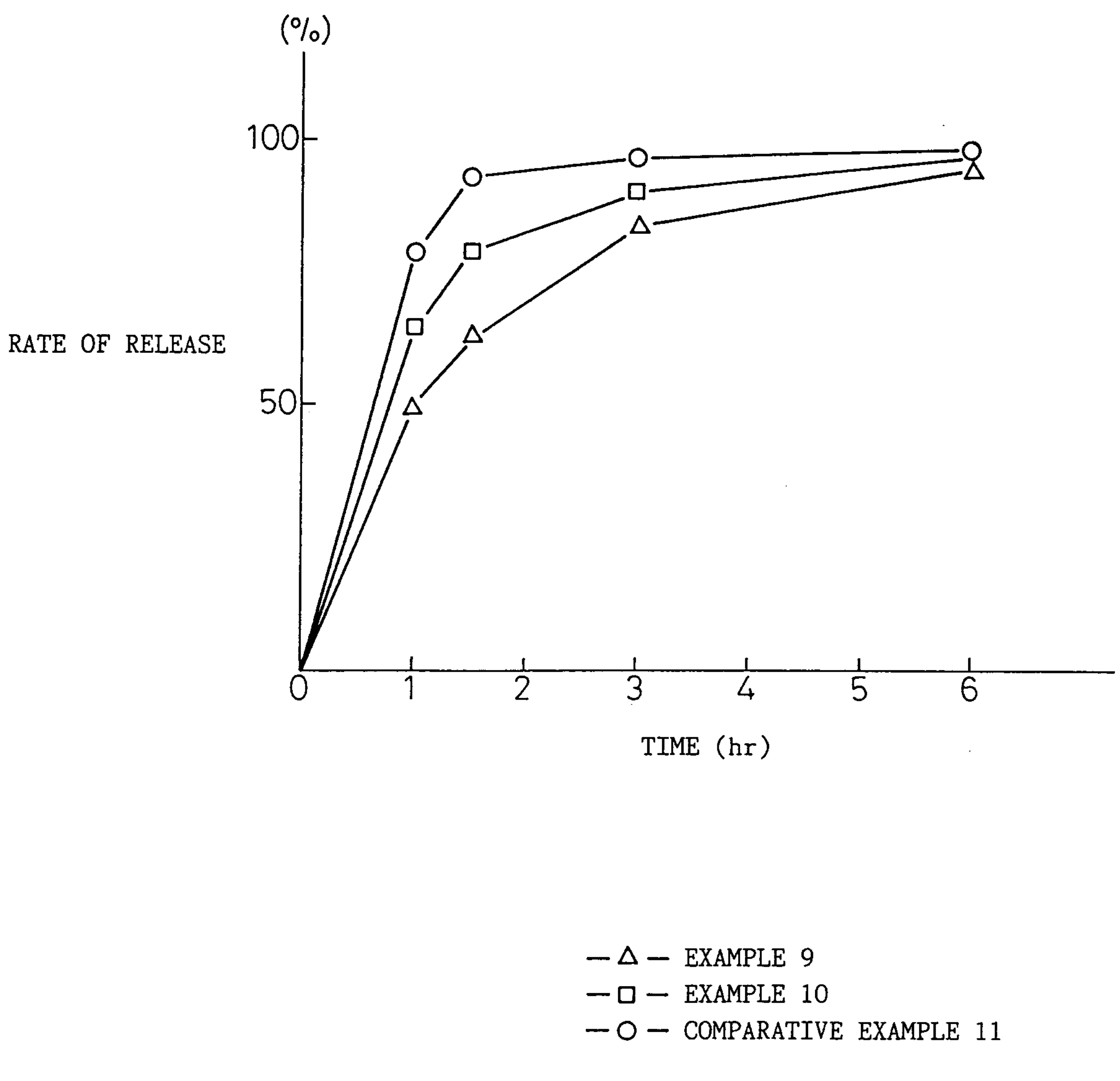
(%)
100
50
RATE OF RELEASE
0
1
2
3
4
5
6
TIME (hr)
—△— EXAMPLE 9
—□— EXAMPLE 10
—○— COMPARATIVE EXAMPLE 11

Fig.4

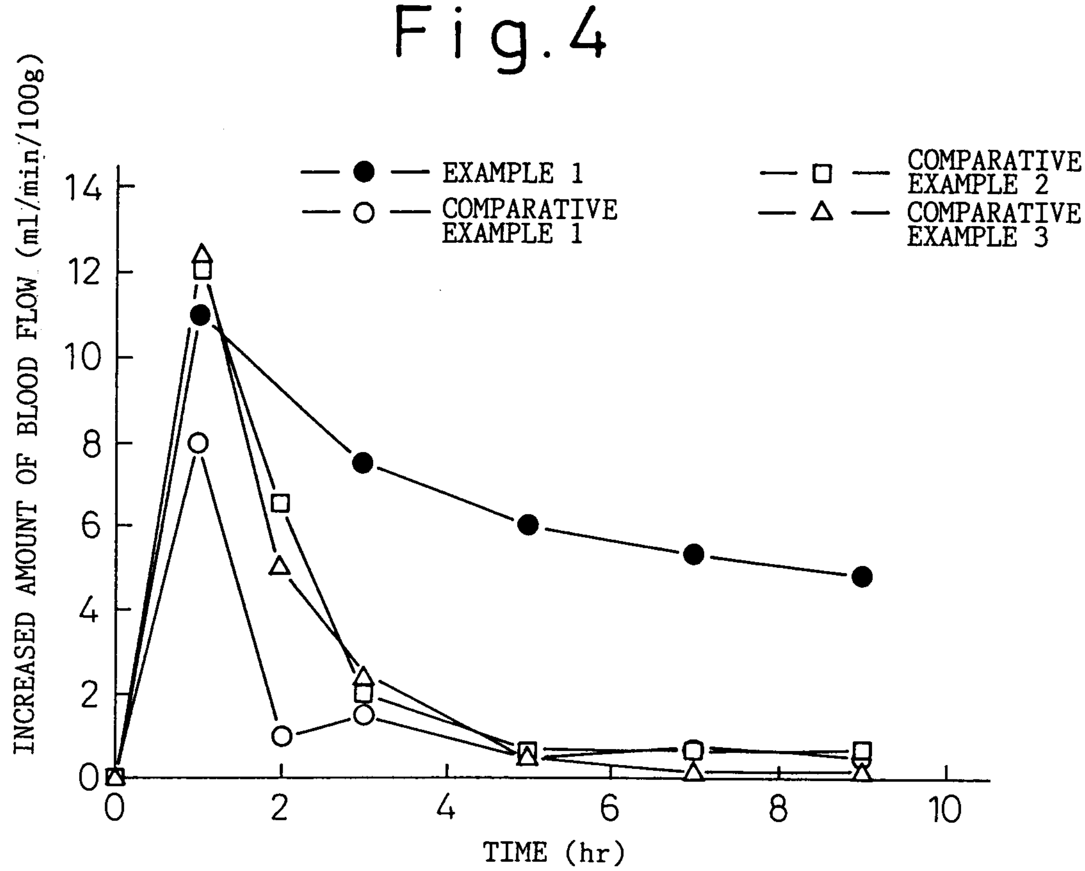
INCREASED AMOUNT OF BLOOD FLOW (ml/min/100g)
14
12
10
8
6
4
2
0
0
2
4
6
8
10
TIME (hr)
EXAMPLE 1
COMPARATIVE EXAMPLE 1
COMPARATIVE EXAMPLE 2
COMPARATIVE EXAMPLE 3

Fig.5

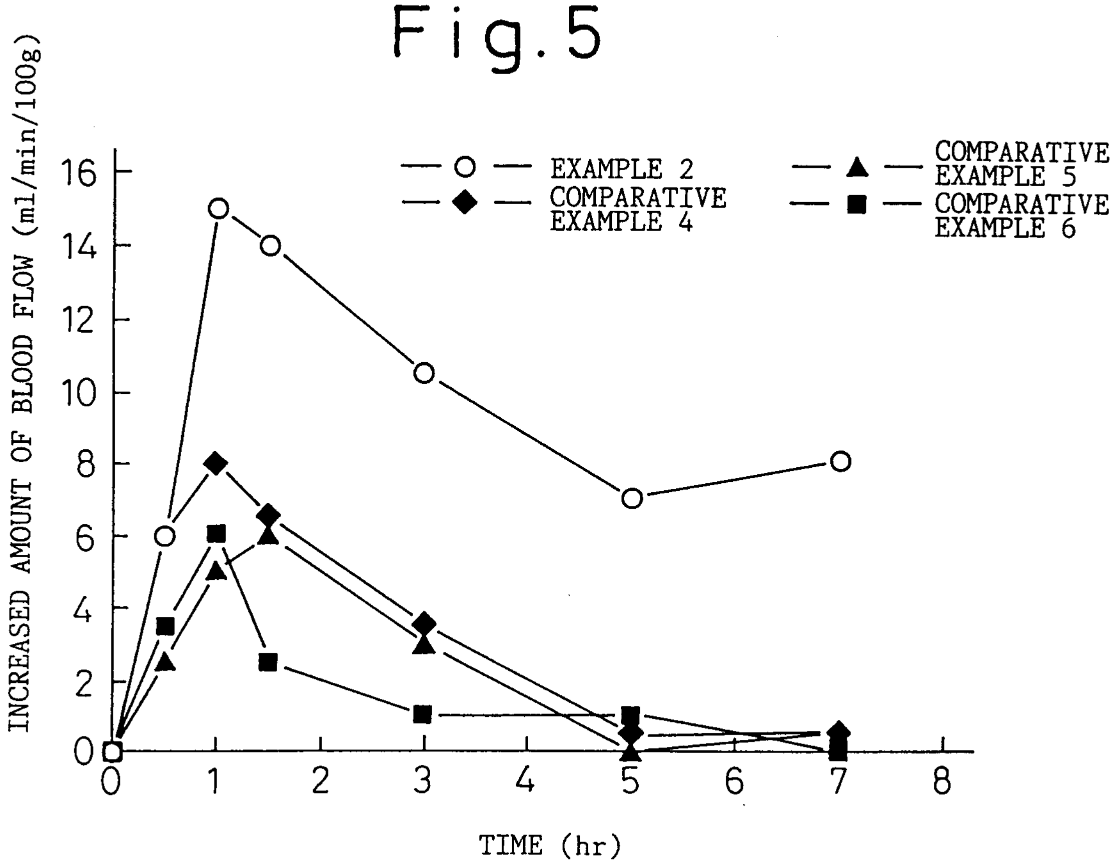
INCREASED AMOUNT OF BLOOD FLOW (ml/min/100g)
16
14
12
10
8
6
4
2
0
0
1
2
3
4
5
6
7
8
TIME (hr)
EXAMPLE 2
COMPARATIVE EXAMPLE 4
COMPARATIVE EXAMPLE 5
COMPARATIVE EXAMPLE 6

Fig.6

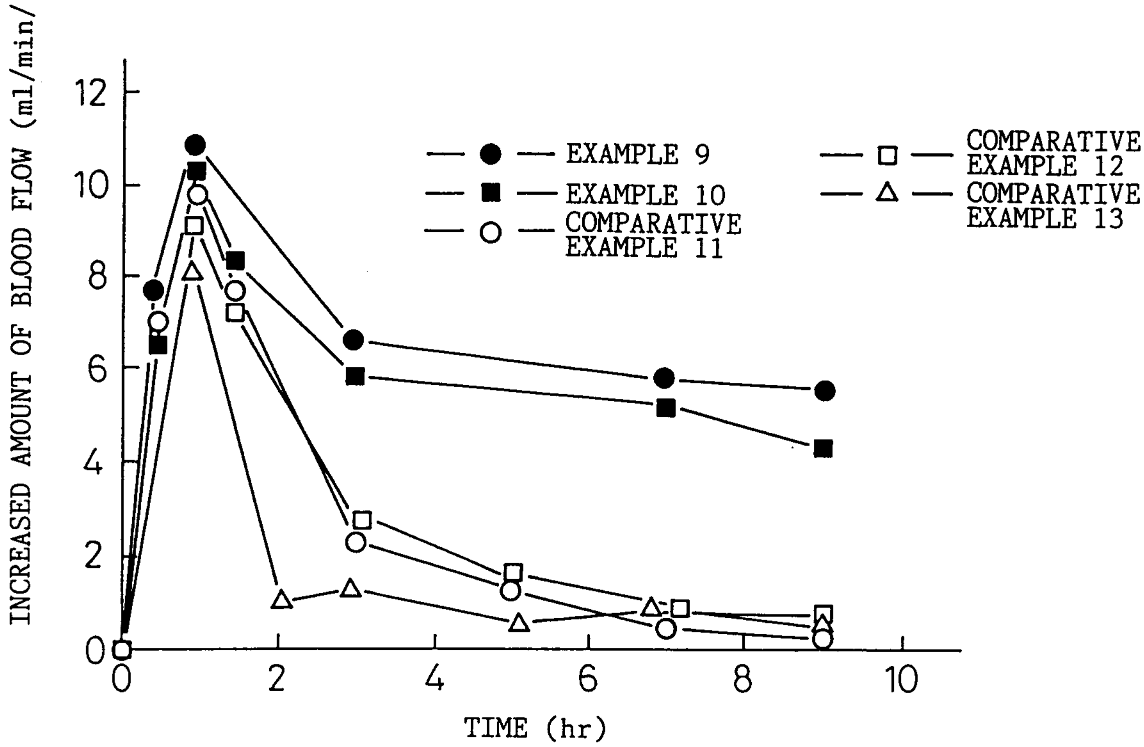
INCREASED AMOUNT OF BLOOD FLOW (ml/min/100g)
12
10
8
6
4
2
0
0
2
4
6
8
10
TIME (hr)
EXAMPLE 9
EXAMPLE 10
COMPARATIVE EXAMPLE 11
COMPARATIVE EXAMPLE 12
COMPARATIVE EXAMPLE 13

Fig.7

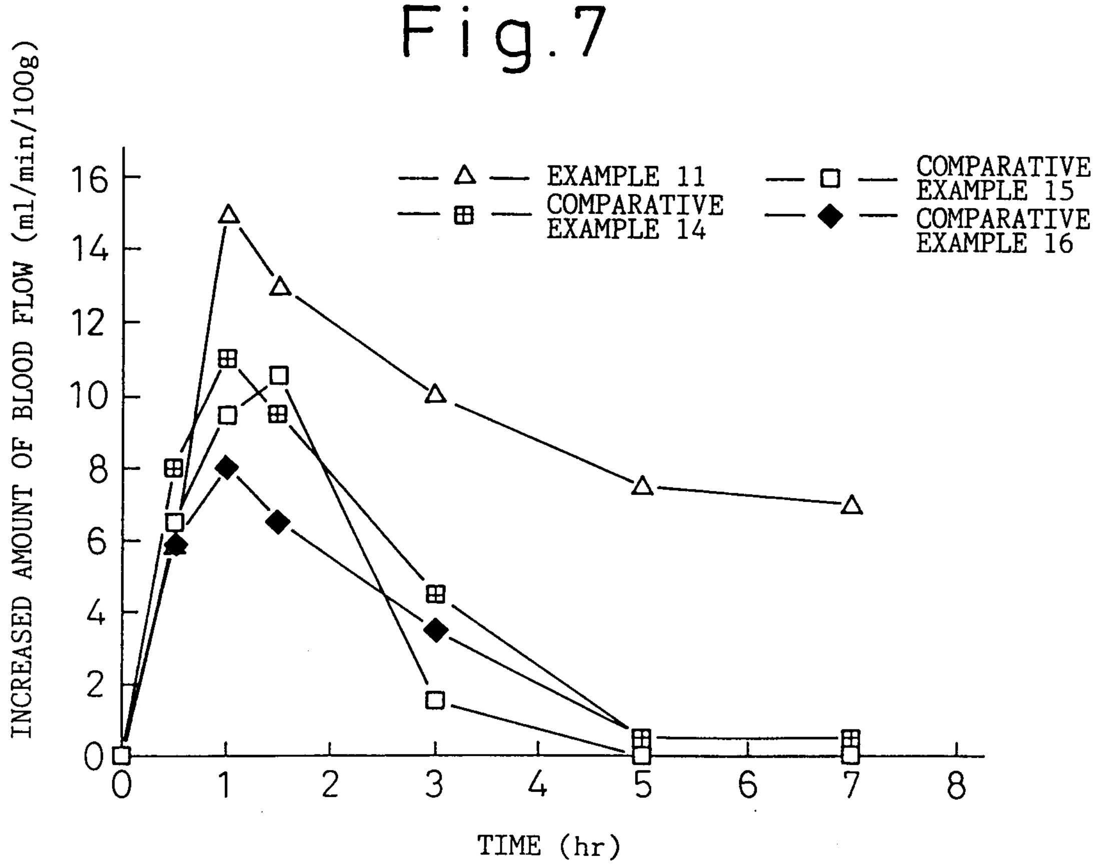
INCREASED AMOUNT OF BLOOD FLOW (ml/min/100g)
16
14
12
10
8
6
4
2
0
0
1
2
3
4
5
6
7
8
TIME (hr)
EXAMPLE 11
COMPARATIVE EXAMPLE 14
COMPARATIVE EXAMPLE 15
COMPARATIVE EXAMPLE 16

## INTERNATIONAL SEARCH REPORT

International application No.
PCT/JP95/00106

**A. CLASSIFICATION OF SUBJECT MATTER**

Int. $Cl^6$ A61K31/557

According to International Patent Classification (IPC) or to both national classification and IPC

**B. FIELDS SEARCHED**

Minimum documentation searched (classification system followed by classification symbols)

Int. $Cl^6$ A61K31/557

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP, A, 4-243827 (Sumitomo Pharmaceuticals Co., Ltd.), August 31, 1992 (31. 08. 92), Claim and examples (Family: none) | 1 - 5 |
| Y | WO, A1, 94/17805 (Fujisawa Pharmaceutical Co., Ltd.), August 18, 1994 (18. 08. 94), Claim & EP, A1, 638312 | 3 - 5 |
| Y | JP, A, 2-207018 (Kao Corp.), August 16, 1990 (16. 08. 90), Claim, lower right column, page 4 to example 4, upper left column, page 5, lower right column, page 5 to examples 11 and 12, upper left column, page 6 (Family: none) | 3 - 5 |
| Y | JP, A, 59-128327 (Teijin Ltd.), Claim, line 16, upper right column to lines 7 to 15, lower right column, line 8, lower left column, page 3, example 2, lower left column, | 3 - 5 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| April 5, 1995 (05. 04. 95) | April 25, 1995 (25. 04. 95) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP95/00106

C (Continuation). DOCUMENTS CONSIDERED TO BE RELEVANT

| Category* | Citation of document, with indication, where appropriate, of the relevant passages | Relevant to claim No. |
|---|---|---|
| | page 4 (Family: none) | |
| Y | JP, A, 63-135333 (Nitto Denki Kogyo K.K., Ono Pharmaceutical Co., Ltd.), Comparative example 3, lines 6 to 20, upper left column, page 10 | 3 - 5 |

Form PCT/ISA/210 (continuation of second sheet) (July 1992)